# EUROPEAN PATENT SPECIFICATION

(11) **EP 3 886 841 B1**
(45) Date of publication and mention of the grant of the patent: **09.09.2026**
(21) Application number: 19824097.0
(22) Date of filing: 26.11.2019
(51) Int. Cl.: A61K 31/404, A61P 1/00

(54) **ETRASIMOD FOR USE IN TREATING ULCERATIVE COLITIS**
ETRASIMOD ZUR VERWENDUNG BEI DER BEHANDLUNG VON COLITIS ULCEROSA
ETRASIMOD POUR UTILISATION DANS LE TRAITEMENT DE LA COLITE ULCÉREUSE

(30) Priority: 30.11.2018 US 201862773936 P; 20.05.2019 US 201962850464 P
(43) Date of publication of application: 06.10.2021
(73) Proprietor: Arena Pharmaceuticals, Inc., New York, NY 10001-2192 (US)
(72) Inventor: NAIK, Snehal, San Diego, California 92130 (US)
(74) Representative: Pfizer
(86) International application number: PCT/US2019/063413
(87) International publication number: WO 2020/112880

(56) References cited:
- WO-A1-2016/112075
- WO-A1-2018/151873
- US-A1- 2013 203 807
- SU HAU-JYUN ET AL: "Inflammatory bowel disease and its treatment in 2018: Global and Taiwanese status updates", JOURNAL OF THE FORMOSAN MEDICAL ASSOCIATION, EXCERPTA MEDICA ASIA, HONG KONG, HK, vol. 118, no. 7, 24 July 2018 (2018-07-24), pages 1083 - 1092, XP085710004, ISSN: 0929-6646, DOI: 10.1016/J.JFMA.2018.07.005
- ANONYMOUS: "Arena Pharmaceuticals Reports Positive Phase 2 Results from the OASIS Trial for Etrasimod in Patients with Ulcerative Colitis", ARENA PHARMACEUTICALS PRESS RELEASES, 19 March 2018 (2018-03-19), XP055617285, Retrieved from the Internet <URL:http://invest.arenapharm.com/node/18666/pdf> [retrieved on 20190902]
- W J SANDBORN ET AL: "UEG Week 2018 Oral Presentations OP242", UNITED EUROPEAN GASTROENTEROLOGY JOURNAL : UEG JOURNAL, vol. 6, no. 8_suppl, 1 October 2018 (2018-10-01), GB, pages A1 - A134, XP055670712, ISSN: 2050-6406, DOI: 10.1177/2050640618792817
- ANONYMOUS: "Etrasimod (APD334), an ooral, nwxt-generation sphingosine-1-hopsphate receptor modulator inhibits the development of colitis in lymphoid-null mice injected with colitogenic CD4+ T cells", 1 April 2017 (2017-04-01), XP055550877, Retrieved from the Internet <URL:https://www.fasebj.org/doi/abs/10.1096/fasebj.31.1_supplement.993.11> [retrieved on 20190201]
- FISCHER S ET AL: "What rheumatologists can learn from gastroenterologists", ZEITSCHRIFT FÜR RHEUMATOLOGIE, SPRINGER, DE, vol. 77, no. 6, 4 June 2018 (2018-06-04), pages 460 - 468, XP036564670, ISSN: 0340-1855, [retrieved on 20180604], DOI: 10.1007/S00393-018-0482-X

## Description

Provided is a compound for use in methods useful in the treatment of: sphingosine 1-phosphate subtype 1 (S1P₁ or SIP1) receptor-associated disorders.

The sphingosine-1-phosphate (S1P) receptors 1-5 constitute a family of G protein-coupled receptors with a seven-transmembrane domain. These receptors, referred to as S1P₁ to S1P₅ (formerly termed endothelial differentiation gene (EDG) receptor-1, -5, -3, -6, and -8, respectively; Chun et al., Pharmacological Reviews, 54:265-269, 2002), are activated *via* binding by sphingosine-1-phosphate, which is produced by the sphingosine kinase-catalyzed phosphorylation of sphingosine. S1P₁, S1P₄, and S1P₅ receptors activate Gi but not Gq, whereas S1P₂ and S1P₃ receptors activate both Gi and Gq. The S1P₃ receptor, but not the S1P₁ receptor, responds to an agonist with an increase in intracellular calcium.

In view of the growing demand for S1P₁ agonists useful in the treatment of S1P₁ receptor-associated disorders, the compound (*R*)-2-(7-(4-cyclopentyl-3-(trifluoromethyl)benzyloxy)-1,2,3,4-tetrahydrocyclopenta[b]indol-3-yl)acetic acid (Compound 1, APD334) , or a pharmaceutically acceptable salt, solvate, or hydrate thereof, 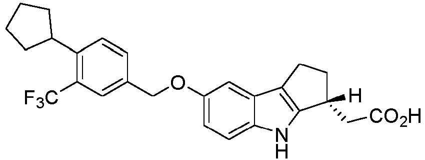 has emerged as an important new compound, see PCT patent application, Serial No. PCT/US2009/004265. Compound 1, or a pharmaceutically acceptable salt, solvate, or hydrate thereof, is an investigational drug candidate intended for the treatment of sphingosine 1-phosphate subtype 1 (S1P₁) receptor-associated disorders.

According to international patent publication WO-2016/112075, Compound 1, or a pharmaceutically acceptable salt, hydrate or solvate thereof, can be used to treat an S1P₁ receptor-associated disorder, such as ulcerative colitis, by administering an amount equivalent to about 1.5 to 2.5 mg of compound 1.

Many S1P₁ agonists cause side effects, and particularly cardiovascular related adverse events, that require that doctors titrate patients slowly to a maintenance dose. This titration period can take weeks or even a month. The complexity and length of the titration regimen may result in prematurely discontinuing therapy by patients prior to reaching the maintenance dose or to doctors preferring other therapeutic options.

There exists a need for effectively treating individuals who are in need of treatment with Compound 1, or a pharmaceutically acceptable salt, solvate, or hydrate thereof, and more particularly, methods for determining if an individual may be a responder to such treatment. The present disclosure satisfies this need and provides related advantages as well.

Citation of any reference throughout this application is not to be construed as an admission that such reference is prior art to the present application.

### SUMMARY

Provided is the compound (*R*)-2-(7-(4-cyclopentyl-3-(trifluoromethyl)benzyloxy)-1,2,3,4-tetrahydrocyclopenta[b]indol-3-yl)acetic acid (Compound 1), or a pharmaceutically acceptable salt, solvate or hydrate thereof,
for use in a method for the treatment of a sphingosine 1-phosphate subtype 1 (S1P₁) receptor-associated disorder in an individual in need thereof, wherein the S1P₁ receptor-associated disorder is ulcerative colitis, comprising:
administering Compound 1, or a pharmaceutically acceptable salt, solvate or hydrate thereof, to the individual for a first time period, wherein the first time period of treatment is 4 weeks of treatment;
wherein the dose is in an amount equivalent to 2 mg of Compound 1;
wherein the dose of Compound 1, or a pharmaceutically acceptable salt, hydrate, or solvate thereof is administered once daily to the individual;
wherein the Compound 1, or a pharmaceutically acceptable salt, hydrate, or solvate thereof, is administered orally; and
continuing administration of Compound 1 for a second time period if the individual achieves at least 1, 2, 3, 4 ,5, 6, 7, 8, 9, 10, 11, 12, 13, 14, 15, 16, 17, 18, 19, 20, 21, 22, 23, 24, 25, 30, 35, 40, 45, 50, 55, 60, 65, 70, 75, 80, 85, 90, or 95% reduction in fecal calprotectin by the first time period.

### BRIEF DESCRIPTION OF THE DRAWINGS

**Figure 1** shows observed lymphocyte counts (10³/µL) in the safety population.
**Figure 2** shows observed neutrophil counts (10³/µL) in the safety population.
**Figure 3** shows electrocardiogram (ECG) heart rate (bpm) as change from baseline by time point in the safety population.
**Figure 4** shows a comparison of percentage of patients with endoscopic improvement for etrasimod (the L-arginine salt of Compound 1), ozanimod, XELJANZ^{®} (tofacitinib citrate), ENTYVIO^{®} (vedolizumab), SIMPONI^{®} (golimumab), and HUMIRA^{®} (adalimumab).
**Figure 5** shows a comparison of percentage of patients in clinical remission, defined as the proportion of patients with total Mayo score ≤ 2 points and no subscore >1, for etrasimod (the L-arginine salt of Compound 1), ozanimod, XELJANZ^{®} (tofacitinib citrate), ENTYVIO^{®} (vedolizumab), SIMPONI^{®} (golimumab), and HUMIRA^{®} (adalimumab).
**Figures 6A-B** show the effect of placebo, etrasimod 1 mg, and etrasimod 2 mg on (A) fecal calprotectin and **(B)** C-reactive protein levels over time. *P* values versus placebo: * *P*≤0.05, ** *P*≤0.001. BL, baseline; CRP, C-reactive protein; IQR, interquartile range; Wk, week.
**Figures 7A-B** show the effect of etrasimod 2 mg on **(A)** fecal calprotectin and **(B)** C-reactive protein over time in patients achieving and not achieving remission at week 12. P values compare patients who achieved remission vs patients who did not achieve remission: * *P*≤0.005, ** *P*≤0.0001. BL, baseline; CRP, C-reactive protein; IQR, interquartile range; Wk, week.

### DETAILED DESCRIPTION

As used in the present specification, the following words and phrases are generally intended to have the meanings as set forth below, except to the extent that the context in which they are used indicates otherwise.

**COMPOUND 1:** As used herein, "Compound 1" means (*R*)-2-(7-(4-cyclopentyl-3-(trifluoromethyl)benzyloxy)-1,2,3,4-tetrahydrocyclopenta[*b*]indol-3-yl)acetic acid including crystalline forms thereof. As a non-limiting example, Compound 1 may be present as an anhydrous, non-solvated crystalline form as described in WO 2010/011316. As another non-limiting example, an L-arginine salt of Compound 1 may be present as an anhydrous, non-solvated crystalline form as described in WO 2010/011316 and WO 2011/094008. As another non-limiting example, a calcium salt of Compound 1 may be present as a crystalline form as described in WO 2010/011316.

**ADMINISTERING:** As used herein, "administering" means to provide a compound or other therapy, remedy, or treatment such that an individual internalizes a compound.

**PRESCRIBING:** As used herein, "prescribing" means to order, authorize, or recommend the use of a drug or other therapy, remedy, or treatment. In some embodiments, a health care practitioner can orally advise, recommend, or authorize the use of a compound, dosage regimen or other treatment to an individual. In this case the health care practitioner may or may not provide a prescription for the compound, dosage regimen, or treatment. Further, the health care practitioner may or may not provide the recommended compound or treatment. For example, the health care practitioner can advise the individual where to obtain the compound without providing the compound. In some embodiments, a health care practitioner can provide a prescription for the compound, dosage regimen, or treatment to the individual. For example, a health care practitioner can give a written or oral prescription to an individual. A prescription can be written on paper or on electronic media such as a computer file, for example, on a handheld computer device. For example, a health care practitioner can transform a piece of paper or electronic media with a prescription for a compound, dosage regimen, or treatment. In addition, a prescription can be called in (oral), faxed in (written), or submitted electronically via the internet to a pharmacy or a dispensary. In some embodiments, a sample of the compound or treatment can be given to the individual. As used herein, giving a sample of a compound constitutes an implicit prescription for the compound. Different health care systems around the world use different methods for prescribing and/or administering compounds or treatments and these methods are encompassed by the disclosure.

A prescription can include, for example, an individual's name and/or identifying information such as date of birth. In addition, for example, a prescription can include: the medication name, medication strength, dose, frequency of administration, route of administration, number or amount to be dispensed, number of refills, physician name, physician signature, and the like. Further, for example, a prescription can include a DEA number and/or state number.

A healthcare practitioner can include, for example, a physician, nurse, nurse practitioner, or other related health care professional who can prescribe or administer compounds (drugs) for the treatment of a sphingosine 1-phosphate subtype 1 (S1P₁) receptor-associated disorder. In addition, a healthcare practitioner can include anyone who can recommend, prescribe, administer, or prevent an individual from receiving a compound or drug including, for example, an insurance provider.

**PREVENT, PREVENTING, OR PREVENTION:** As used herein, the term "prevent," "preventing", or "prevention" such as prevention of a sphingosine 1-phosphate subtype 1 (S1P₁) receptor-associated disorder or the occurrence or onset of one or more symptoms associated with the particular disorder and does not necessarily mean the complete prevention of the disorder. For example, the term "prevent," "preventing" and "prevention" means the administration of therapy on a prophylactic or preventative basis to an individual who may ultimately manifest at least one symptom of a disease or condition but who has not yet done so. Such individuals can be identified on the basis of risk factors that are known to correlate with the subsequent occurrence of the disease. Alternatively, prevention therapy can be administered without prior identification of a risk factor, as a prophylactic measure. Delaying the onset of at least one symptom can also be considered prevention or prophylaxis.

**TREAT, TREATING, OR TREATMENT:** As used herein the term "treat," "treating", or "treatment" means the administration of therapy to an individual who already manifests at least one symptom of a disease or condition or who has previously manifested at least one symptom of a disease or condition. For example, "treating" can include alleviating, abating or ameliorating a disease or condition symptoms, preventing additional symptoms, ameliorating the underlying metabolic causes of symptoms, inhibiting the disease or condition, e.g., arresting the development of the disease or condition, relieving the disease or condition, causing regression of the disease or condition, relieving a condition caused by the disease or condition, or stopping the symptoms of the disease or condition. For example, the term "treating" in reference to a disorder means a reduction in severity of one or more symptoms associated with that particular disorder. Therefore, treating a disorder does not necessarily mean a reduction in severity of all symptoms associated with a disorder and does not necessarily mean a complete reduction in the severity of one or more symptoms associated with a disorder.

**TOLERATE:** As used herein, an individual is said to "tolerate" a dose of a compound if administration of that dose to that individual does not result in an unacceptable adverse event or an unacceptable combination of adverse events. One of skill in the art will appreciate that tolerance is a subjective measure and that what may be tolerable to one individual may not be tolerable to a different individual. For example, one individual may not be able to tolerate headache, whereas a second individual may find headache tolerable but is not able to tolerate vomiting, whereas for a third individual, either headache alone or vomiting alone is tolerable, but the individual is not able to tolerate the combination of headache and vomiting, even if the severity of each is less than when experienced alone.

**ADVERSE EVENT:** As used herein, an "adverse event" is an untoward medical occurrence that is associated with treatment with Compound 1 or a pharmaceutically acceptable salt, solvate, or hydrate thereof. In one embodiment, an adverse event is selected from: leukopenia, constipation, diarrhea, nausea, abdominal pain, neutropenia, vomiting, back pain, and menstrual disorder. In one embodiment, an adverse event is heart block, for example, a first degree atrioventricular heart block. In one embodiment, an adverse event is an acute heart rate reduction. In one embodiment, an adverse event is an abnormal pulmonary function test finding, such as an FEV1 below 80%, FVC. In one embodiment, an adverse event is an abnormal liver function test, such as an elevated ALT & AST>2X ULN. In one embodiment, an adverse event is macular edema.

**IN NEED OF TREATMENT** and **IN NEED THEREOF:** As used herein, "in need of treatment" and "in need thereof' when referring to treatment are used interchangeably to mean a judgment made by a caregiver (*e.g.* physician, nurse, nurse practitioner, *etc.* in the case of humans; veterinarian in the case of animals, including non-human mammals) that an individual or animal requires or will benefit from treatment. This judgment is made based on a variety of factors that are in the realm of a caregiver's expertise, but that includes the knowledge that the individual or animal is ill, or will become ill, as the result of a disease, condition or disorder that is treatable by the compounds of the invention. Accordingly, the compounds of the invention can be used in a protective or preventive manner; or compounds of the invention can be used to alleviate, inhibit or ameliorate the disease, condition or disorder.

**INDIVIDUAL:** As used herein, "individual" means any animal, including mammals, preferably mice, rats, other rodents, rabbits, dogs, cats, swine, cattle, sheep, horses, or primates and most preferably humans. In some embodiments, a human individual is referred to a "patient."

**ACUTE zHEART RATE REDUCTION:** As used herein, "acute heart rate reduction" means a heart rate decrease from normal sinus rhythm of, for example, 10 or more beats per minute (bpm), such as less than about 5 bpm, *e.g.,* less than about 4 bpm or less than about 3 bpm or less than 2 bpm, that is maximal within a few hours, for example 1-3 hours, after drug administration, and thereafter the heart rate returns towards the pre-dose value.

**NORMAL SINUS RHYTHM:** As used herein, "normal sinus rhythm" means the sinus rhythm of the individual when not undergoing treatment. The evaluation of normal sinus rhythm is within the ability of a physician. A normal sinus rhythm will generally give rise to a heart rate in the range from 60-100 bpm.

**DOSE:** As used herein, "dose" means a quantity of Compound 1, or a pharmaceutically acceptable salt, solvate, or hydrate thereof, given to the individual for treating or preventing the disease or disorder at one specific time.

**FASTED INDIVIDUAL:** As used herein, "fasted individual" means an individual who has not eaten any food, *i.e.,* has fasted for at least 6-8 hours, such as about 8 hours, before the administration of Compound 1, or a pharmaceutically acceptable salt, hydrate, or solvate thereof, and who does not eat any food and continues to fast for at least 1 hour after the administration of Compound 1, or a pharmaceutically acceptable salt, hydrate, or solvate thereof. In certain embodiments, the individual may also refrain from ingesting certain non-food substances during the fasting period. For example, in certain embodiments the individual does not ingest any supplements and/or drugs during the fasting period. In certain embodiments, the individual does not ingest any high calorie liquids during the fasting period. In certain embodiments, the individual does not ingest any liquids other than water during the fasting period. In certain embodiments, the individual may ingest small amounts of low calorie beverages, such as tea, coffee, or diluted juices.

**MAYO CLINIC SCORE (MCS):** As used herein, "Mayo Clinic Score," "Mayo clinic score," "Mayo score," or "MCS" means an instrument designed to measure disease activity of ulcerative colitis and consists of up to 4 subscores: stool frequency, rectal bleeding, findings of flexible proctosigmoidoscopy, and physician global assessment with each component ranging from 0 to 3 (0=normal, 1=mild, 2=moderate, 3=severe). Total score therefore ranges from 0 to 12, with a higher score indicating more severe disease. The 6-point Mayo score is based on stool frequency and rectal bleeding PROs collected daily using electronic patient diaries and excludes the findings on endoscopy and the physician's global assessment. The 3-point Mayo score is based on stool frequency, rectal bleeding, and findings on endoscopy and has a total score ranging from 0 to 9. The 2-point Mayo score is based on rectal bleeding and findings on endoscopy and has a total score ranging from 0 to 6. The physician's global assessment acknowledges the three other criteria findings of the MCS, the individual's daily record of abdominal discomfort and general sense of well-being, and other observations, such as physical findings and the individual's performance.

**MILDLY TO MODERATELY ACTIVE ULCERATIVE COLITIS:** As used herein, "mildly to moderately active ulcerative colitis" means ulcerative colitis characterized by a 4-component MCS of 4 to 10.

**MODERATELY TO SEVERELY ACTIVE ULCERATIVE COLITIS:** As used herein, "moderately to severely active ulcerative colitis" means ulcerative colitis characterized by a 3-component MCS of 4 to 9 including an endoscopic subscore of ≥ 2 and a rectal bleeding score of ≥ 1. The 3-component MCS uses 3 of the 4 components of the complete MCS (endoscopic findings, rectal bleeding, and stool frequency).

**CLINICAL REMISSION:** As used herein, "clinical remission" with respect to ulcerative colitis means a 3-component Mayo Clinic score as follows: an endoscopy score (using flexible proctosigmoidoscopy) of 0 or 1, a rectal bleeding score of ≤1, and a stool frequency score of 0 or 1 with a decrease of ≥ 1 point from baseline subscore. In some embodiments, clinical remission with respect to ulcerative colitis means a 3-component Mayo Clinic score as follows: an endoscopy score (using flexible proctosigmoidoscopy) of 0 or 1, a rectal bleeding score of 0, and a stool frequency score of 0 or 1 with a decrease of ≥ 1 point from baseline subscore.

**CLINICAL RESPONSE:** As used herein, "clinical response" with respect to ulcerative colitis means a reduction in the 3-component Mayo Clinic score of ≥ 2 points and a decrease of ≥ 30% from baseline with an accompanying decrease in rectal bleeding subscore of ≥ 1 or absolute rectal bleeding score of 0 or 1.

**ENDOSCOPIC IMPROVEMENT:** As used herein, "endoscopic improvement" with respect to ulcerative colitis means ulcerative colitis characterized by a Mayo endoscopic subscore (using findings of flexible proctosigmoidoscopy) of ≤ 1 point.

**ENDOSCOPIC REMISSION:** As used herein, "endoscopic remission" with respect to ulcerative colitis means ulcerative colitis characterized by findings from flexible proctosigmoidoscopy subscore of the Mayo Clinic score = 0.

**IMPROVEMENT IN RECTAL BLEEDING:** As used herein, "improvement in rectal bleeding" with respect to ulcerative colitis means a change from baseline < 0.

**HISTOLOGIC HEALING:** As used herein, "histologic healing" with respect to ulcerative colitis means a score of < 3.1 on the Geboes Index.

**HISTOLOGIC REMISSION:** As used herein, "histologic remission" or "histological remission" with respect to ulcerative colitis means a score of < 2.0 on the Geboes Index.

**MUCOSAL HEALING:** As used herein, "mucosal healing" is both endoscopic improvement and histological remission.

**IMPROVEMENT IN STOOL FREQUENCY:** As used herein, "improvement in stool frequency" with respect to ulcerative colitis means a change from baseline < 0.

**5-AMINOSALICYLATES:** As used herein, "5-aminosalicylates", means a class of drugs that include, for example, CANASA^{®} (mesalamine), COLAZAL^{®} (balsalazide disodium), ASACOL^{®} (mesalamine), DELZICOL^{®} (mesalamine), and DIPENTUM^{®} (olsalazine).

**IMMUNOSUPPRESSIVES:** As used herein, "immunosuppressives", means a class of drugs that include, for example, AZASAN^{®} (Azathioprine), IMURAN^{®} (Azathioprine), GENGRAF^{®} (Cyclosporine), NEORAL^{®} (Cyclosporine), and SANDIMMUNE^{®} (Cyclosporine).

**GLUCOCORTICOSTEROIDS:** As used herein, "glucocorticosteroids", means a class of drugs that include, for example, UCERIS^{®} (budesonide); DELTASONE^{®} (prednisone), MEDROL^{®} (methylprednisolone), and hydrocortisone.

**TNFα ANTAGONISTS:** As used herein, "TNFα antagonists" or "tumor necrosis factor-a antagonists", means a class of drugs that include, for example, SIMPONI^{®} (golimumab), REMICADE^{®} (infliximab), and HUMIRA^{®} (adalimumab).

**INTEGRIN RECEPTOR ANTAGONISTS:** As used herein, "integrin receptor antagonists", means a class of drugs that include, for example, ENTYVIO^{®} (vedolizumab).

**PHARMACEUTICAL COMPOSITION:** As used here, "pharmaceutical composition" means a composition comprising at least one active ingredient, such as Compound 1; including but not limited to, salts, solvates, and hydrates of Compound 1, whereby the composition is amenable to investigation for a specified, efficacious outcome in a mammal (for example, without limitation, a human). Those of ordinary skill in the art will understand and appreciate the techniques appropriate for determining whether an active ingredient has a desired efficacious outcome based upon the needs of the artisan.

**AGONIST:** As used herein, "agonist" means a moiety that interacts with and activates a G-protein-coupled receptor, such as the S1P₁ receptor, such as can thereby initiate a physiological or pharmacological response characteristic of that receptor. For example, an agonist activates an intracellular response upon binding to the receptor, or enhances GTP binding to a membrane. In certain embodiments, an agonist of the invention is an S1P₁ receptor agonist that is capable of facilitating sustained S1P₁ receptor internalization (see *e.g.,* Matloubian et al., Nature, 427, 355, 2004).

**ANTAGONIST:** As used herein, "antagonist" means a moiety that competitively binds to the receptor at the same site as an agonist (for example, the endogenous ligand), but which does not activate the intracellular response initiated by the active form of the receptor and can thereby inhibit the intracellular responses by an agonist or partial agonist. An antagonist does not diminish the baseline intracellular response in the absence of an agonist or partial agonist.

**HYDRATE:** As used herein, "hydrate" means a compound of the invention or a salt thereof, that further includes a stoichiometric or non-stoichiometric amount of water bound by non-covalent intermolecular forces.

**SAFETY POPULATION:** As used herein, "safety population" means all randomized subjects who received study medication.

**SOLVATE:** As used herein, "solvate" means a compound of the invention or a salt, thereof, that further includes a stoichiometric or non-stoichiometric amount of a solvent bound by non-covalent intermolecular forces. Preferred solvents are volatile, non-toxic, and/or acceptable for administration to humans in trace amounts.

The compounds according to the invention may optionally exist as pharmaceutically acceptable salts including pharmaceutically acceptable acid addition salts prepared from pharmaceutically acceptable non-toxic acids including inorganic and organic acids. Representative acids include, but are not limited to, acetic, benzenesulfonic, benzoic, camphorsulfonic, citric, ethenesulfonic, dichloroacetic, formic, fumaric, gluconic, glutamic, hippuric, hydrobromic, hydrochloric, isethionic, lactic, maleic, malic, mandelic, methanesulfonic, mucic, nitric, oxalic, pamoic, pantothenic, phosphoric, succinic, sulfiric, tartaric, oxalic, p-toluenesulfonic and the like, such as those pharmaceutically acceptable salts listed by Berge et al., Journal of Pharmaceutical Sciences, 66:1-19 (1977).

The acid addition salts may be obtained as the direct products of compound synthesis. In the alternative, the free base may be dissolved in a suitable solvent containing the appropriate acid and the salt isolated by evaporating the solvent or otherwise separating the salt and solvent. The compounds of this invention may form solvates with standard low molecular weight solvents using methods known to the skilled artisan.

It is understood that when the phrase "pharmaceutically acceptable salts, solvates and hydrates" or the phrase "pharmaceutically acceptable salt, solvate, or hydrate" is used when referring to Compound 1, it embraces pharmaceutically acceptable solvates and/or hydrates of Compound 1, pharmaceutically acceptable salts of Compound 1, as well as pharmaceutically acceptable solvates and/or hydrates of pharmaceutically acceptable salts of Compound 1. It is also understood that when the phrase "pharmaceutically acceptable solvates and hydrates" or the phrase "pharmaceutically acceptable solvate or hydrate" is used when referring to Compound 1that are salts, it embraces pharmaceutically acceptable solvates and/or hydrates of such salts.

It will be apparent to those skilled in the art that the dosage forms described herein may comprise, as the active component, either Compound 1 or a pharmaceutically acceptable salt or as a solvate or hydrate thereof. Moreover, various hydrates and solvates of Compound 1 and their salts will find use as intermediates in the manufacture of pharmaceutical compositions. Typical procedures for making and identifying suitable hydrates and solvates, outside those mentioned herein, are well known to those in the art; see for example, pages 202-209 of K.J. Guillory, "Generation of Polymorphs, Hydrates, Solvates, and Amorphous Solids," in: Polymorphism in Pharmaceutical Solids, ed. Harry G. Britain, Vol. 95, Marcel Dekker, Inc., New York, 1999. Accordingly, one aspect of the present disclosure pertains to methods of prescribing and/or administering hydrates and solvates of Compound 1 and/or its pharmaceutical acceptable salts, that can be isolated and characterized by methods known in the art, such as, thermogravimetric analysis (TGA), TGA-mass spectroscopy, TGA-Infrared spectroscopy, powder X-ray diffraction (XRPD), Karl Fisher titration, high resolution X-ray diffraction, and the like. There are several commercial entities that provide quick and efficient services for identifying solvates and hydrates on a routine basis. Example companies offering these services include Wilmington PharmaTech (Wilmington, DE), Avantium Technologies (Amsterdam) and Aptuit (Greenwich, CT).

The present disclosure includes all isotopes of atoms occurring in the present compounds, salts, solvates, and hydrates. Isotopes include those atoms having the same atomic number but different mass numbers. One aspect of the present invention includes every combination of one or more atoms in the present compounds, salts, solvates, and hydrates that is replaced with an atom having the same atomic number but a different mass number. One such example is the replacement of an atom that is the most naturally abundant isotope, such as ¹H or ¹²C, found in one the present compounds, salts, solvates, and hydrates, with a different atom that is not the most naturally abundant isotope, such as ²H or ³H (replacing ¹H), or ¹¹C, ¹³C, or ¹⁴C (replacing ¹²C). When such a replacement has taken place, it is commonly referred to as being isotopically-labeled. Isotopic-labeling of the present compounds, salts, solvates, and hydrates can be accomplished using any one of a variety of different synthetic methods know to those of ordinary skill in the art and they are readily credited with understanding the synthetic methods and available reagents needed to conduct such isotopic-labeling. By way of general example, and without limitation, isotopes of hydrogen include ²H (deuterium) and ³H (tritium). Isotopes of carbon include ¹¹C, ¹³C, and ¹⁴C. Isotopes of nitrogen include ¹³N and ¹⁵N. Isotopes of oxygen include ¹⁵O, ¹⁷O, and ¹⁸O. An isotope of fluorine includes ¹⁸F. An isotope of sulfur includes ³⁵S. An isotope of chlorine includes ³⁶Cl. Isotopes of bromine include ⁷⁵Br, ⁷⁶Br, ⁷⁷Br, and ⁸²Br. Isotopes of iodine include ¹²³I, ¹²⁴I, ¹²⁵I, and ¹³¹I. Another aspect of the present invention includes compositions, such as, those prepared during synthesis, preformulation, and the like, and pharmaceutical compositions, such as, those prepared with the intent of using in a mammal for the treatment of one or more of the disorders described herein, comprising one or more of the present compounds, salts, solvates, and hydrates, wherein the naturally occurring distribution of the isotopes in the composition is perturbed. Another aspect of the present invention includes compositions and pharmaceutical compositions comprising the compounds, salts, solvates, and hydrates, as described herein wherein the salt is enriched at one or more positions with an isotope other than the most naturally abundant isotope. Methods are readily available to measure such isotope perturbations or enrichments, such as, mass spectrometry, and for isotopes that are radio-isotopes additional methods are available, such as, radio-detectors used in connection with HPLC or GC.

Compounds of the present invention can be converted to "prodrugs." The term "prodrugs" means compounds that have been modified with specific chemical groups known in the art and that when administered into an individual undergo biotransformation to give the parent compound. Prodrugs can thus be viewed as compounds of the invention containing one or more specialized non-toxic protective groups used in a transient manner to alter or to eliminate a property of the compound. In one general aspect, the "prodrug" approach is utilized to facilitate oral absorption. A thorough discussion is provided in T. Higuchi and V. Stella, Prodrugs as Novel Delivery Systems Vol. 14 of the A.C.S. Symposium Series; and in Bioreversible Carriers in Drug Design, ed. Edward B. Roche, American Pharmaceutical Association and Pergamon Press, 1987.

Throughout this specification, unless the context requires otherwise, the word "comprise", or variations such as "comprises" or "comprising" will be understood to imply the inclusion of a stated step or element or integer or group of steps or elements or integers but not the exclusion of any other step or element or integer or group of elements or integers.

The present invention(s) is not to be limited in scope by the specific embodiments described herein, which are intended for the purpose of exemplification only. Functionally-equivalent products, compositions, and methods are clearly within the scope of the invention(s), as described herein.

Responsiveness is measured by a reduction in fecal calprotectin level.

In some embodiments, responsiveness is measured by a fecal calprotectin level of less than about 1000, 975, 950, 925, 900, 875, 850, 825, 800, 775, 750, 725, 700, 675, 650, 625, 600, 575, 550, 525, 500, 475, 450, 425, 400, 375, 350, 325, 300, 275, 250, 200, 175, 174, 173, 172, 171, 170, 169, 168, 167, 166, 165, 164, 163, 162, 161, 160, 159, 158, 157, 156, 155, 154, 153, 152, 151,150, 149, 148, 147, 146, 145, 144, 143, 142, 141, 140, 139, 138, 137, 136, 135, 134, 133, 132, 131, 130, 129, 128, 127, 126, 125, 124, 123, 122, 121, 120, 119, 118, 117, 116, 114, 113, 112, 111, 110, 109, 108, 107, 106, 105, 104, 103, 102, 101, 100, 99 ,98, 97, 96, 95, 94, 93, 92, 91, 90, 89, 88, 87, 86, 85, 84, 83, 82, 81, 80, 79, 78, 77, 76, 75, 74, 73, 72, 71, 70, 69, 68, 67, 66, 65, 64, 63, 62, 61, 60, 59, 58, 57, 56, 55, 54, 53, 52, 51, 50, 49, 48, 47, 46, 45, 44, 43, 42, 41, or 40 ug/g.

In some embodiments, the method further comprises monitoring the individual for an active infection.

In some embodiments, the method further comprises discontinuing administration if the individual develops an active infection.

In some embodiments, the active infection is a serious active infection.

In some embodiments, the method further comprises monitoring for adverse events during the administration of Compound 1, or a pharmaceutically acceptable salt, hydrate, or solvate thereof, and optionally, interrupting or terminating the administration of Compound 1, or a pharmaceutically acceptable salt, hydrate, or solvate thereof.

In some embodiments, the treatment further comprises monitoring heart rate during the administration, monitoring pulmonary function during the administration, or monitoring liver function during the administration.

In some embodiments, the treatment further comprises monitoring heart rate during the administration.

In some embodiments, the treatment further comprises monitoring pulmonary function during the administration.

In some embodiments, the treatment further comprises monitoring liver function during the administration.

In some embodiments, the method reduces the incidence and severity of adverse events resulting from the treatment of the sphingosine 1-phosphate subtype 1 (S1P₁) receptor-associated disorder.

In some embodiments, the adverse event is a serious adverse event.

In some embodiments, the serious adverse event is selected from leukopenia, constipation, diarrhea, nausea, abdominal pain, neutropenia, vomiting, back pain, and menstrual disorder.

In some embodiments, the method results in no serious adverse events.

In some embodiments, Compound 1 is administered without causing a reduction of more than 6 bpm in heart rate.

In some embodiments, Compound 1 is administered without a first-dose effect on heart rate as seen with other S1P receptor modulators. In some embodiments, Compound 1 is administered without a first-dose effect on AV conduction as seen with other S1P receptor modulators.

In some embodiments, the individual was previously administered at least one agent selected from: a TNF antagonist, an integrin antagonist, and an immunosuppressive agent.

In some embodiments, the individual was previously administered vedolizumab.

In some embodiments, the individual had an inadequate response with, lost response to, or was intolerant to the at least one agent.

In some embodiments, the individual had demonstrated, over the previous 3 month period, an inadequate response to, loss of response to, or intolerance of at least one agent selected from oral 5-aminosalicylates, corticosteroids, immunosuppressives, TNFα antagonists, and integrin antagonists. In some embodiments, the individual had demonstrated, over the previous 6 month period, an inadequate response to, loss of response to, or intolerance of at least one agent selected from oral 5-aminosalicylates, corticosteroids, immunosuppressives, TNFα antagonists, and integrin antagonists. In some embodiments, the individual had demonstrated, over the previous 9 month period, an inadequate response to, loss of response to, or intolerance of at least one agent selected from oral 5-aminosalicylates, corticosteroids, immunosuppressives, TNFα antagonists, and integrin antagonists. In some embodiments, the individual had demonstrated, over the previous 1 year period, an inadequate response to, loss of response to, or intolerance of at least one agent selected from oral 5-aminosalicylates, corticosteroids, immunosuppressives, TNFα antagonists, and integrin antagonists. In some embodiments, the individual had demonstrated, over the previous 2 year period, an inadequate response to, loss of response to, or intolerance of at least one agent selected from oral 5-aminosalicylates, corticosteroids, immunosuppressives, TNFα antagonists, and integrin antagonists. In some embodiments, the individual had demonstrated, over the previous 3 year period, an inadequate response to, loss of response to, or intolerance of at least one agent selected from oral 5-aminosalicylates, corticosteroids, immunosuppressives, TNFα antagonists, and integrin antagonists. In some embodiments, the individual had demonstrated, over the previous 4 year period, an inadequate response to, loss of response to, or intolerance of at least one agent selected from oral 5-aminosalicylates, corticosteroids, immunosuppressives, TNFα antagonists, and integrin antagonists. In some embodiments, the individual had demonstrated, over the previous 5 year period, an inadequate response to, loss of response to, or intolerance of at least one agent selected from oral 5-aminosalicylates, corticosteroids, immunosuppressives, TNFα antagonists, and integrin antagonists.

In some embodiments, Compound 1, or a pharmaceutically acceptable salt, hydrate, or solvate thereof, is administered without titration.

In some embodiments, the individual has fasted prior to being administered Compound 1, or a pharmaceutically acceptable salt, hydrate, or solvate thereof.

In some embodiments, treating comprises inducing and/or maintaining clinical response; improving endoscopic appearance of the mucosa; and/or inducing and/or maintaining clinical remission.

In some embodiments, prior to the administering the individual has a 3-component Mayo Clinic Score of at least 6.

In some embodiments, the method results in an improvement of the individual's 3-component Mayo Clinic Score. In some embodiments, the method results in an improvement of the individual's 2-component Mayo Clinic Score. In some embodiments, the method results in an improvement of the individual's Total Mayo Clinic Score.

In some embodiments of the method of treatment of ulcerative colitis, such as moderately to severely active ulcerative colitis, the treatment results in endoscopic improvement, e.g., improving endoscopic appearance of the mucosa.

In some embodiments of the method of treatment of ulcerative colitis, such as moderately to severely active ulcerative colitis, the treatment results in inducing clinical remission. In some embodiments of the method of treatment of ulcerative colitis, such as moderately to severely active ulcerative colitis, the treatment results in maintaining clinical remission. In some embodiments of the method of treatment of ulcerative colitis, such as moderately to severely active ulcerative colitis, the treatment results in inducing and maintaining clinical remission.

In some embodiments of the method of treatment of ulcerative colitis, such as moderately to severely active ulcerative colitis, the treatment results in inducing clinical response. In some embodiments of the method of treatment of ulcerative colitis, such as moderately to severely active ulcerative colitis, the treatment results in maintaining clinical response. In some embodiments of the method of treatment of ulcerative colitis, such as moderately to severely active ulcerative colitis, the treatment results in inducing and maintaining clinical response.

In some embodiments, the treatment reduces a lymphocyte count in the individual by at least 40%. In some embodiments, the treatment reduces a lymphocyte count in the individual by at least 45%, 50%, 55%, 60%, or 65%.

In some embodiments of the method of treatment of ulcerative colitis, such as moderately to severely active ulcerative colitis, the treatment results in corticosteroid-free remission.

In some embodiments of the method of treatment of ulcerative colitis, such as moderately to severely active ulcerative colitis, the treatment results in endoscopic remission.

In some embodiments of the method of treatment of ulcerative colitis, such as moderately to severely active ulcerative colitis, the treatment results in an improvement in rectal bleeding.

In some embodiments of the method of treatment of ulcerative colitis, such as moderately to severely active ulcerative colitis, the treatment results in histologic improvement.

In some embodiments of the method of treatment of i ulcerative colitis, such as moderately to severely active ulcerative colitis, the treatment results in histologic healing.

In some embodiments of the method of treatment of ulcerative colitis, such as moderately to severely active ulcerative colitis, the treatment results in histologic remission.

In some embodiments of the method of treatment of ulcerative colitis, such as moderately to severely active ulcerative colitis, the treatment results in mucosal healing.

In some embodiments of the method of treatment of ulcerative colitis, such as moderately to severely active ulcerative colitis, the treatment results in an improvement in stool frequency.

In some embodiments, treating is reducing a sign and/or symptom of ulcerative colitis. In some embodiments, treating is reducing a sign of ulcerative colitis. In some embodiments, treating is reducing a symptom of ulcerative colitis.

In some embodiments, treating is inducing and/or maintaining clinical remission. In some embodiments, treating is inducing and maintaining clinical remission. In some embodiments, treating is inducing and/or maintaining clinical remission and/or clinical response. In some embodiments, treating is inducing and maintaining clinical remission and clinical response. In some embodiments, treating is inducing clinical remission and/or clinical response. In some embodiments, treating is maintaining clinical remission and/or clinical response. In some embodiments, treating is inducing clinical remission and clinical response. In some embodiments, treating is maintaining clinical remission and clinical response. In some embodiments, treating is inducing and/or maintaining clinical remission and/or mucosal healing. In some embodiments, treating is inducing and maintaining clinical remission and mucosal healing. In some embodiments, treating is inducing and maintaining mucosal healing. In some embodiments, treating is inducing and maintaining clinical remission. In some embodiments, treating is inducing clinical remission. In some embodiments, treating is inducing mucosal healing. In some embodiments, treating is maintaining clinical remission. In some embodiments, treating is maintaining mucosal healing. In some embodiments, treating is achieving and/or sustaining clinical remission in induction responders. In some embodiments, treating is achieving and sustaining clinical remission in induction responders. In some embodiments, treating is achieving clinical remission in induction responders. In some embodiments, treating is sustaining clinical remission in induction responders. In some embodiments, treating is inducing and/or maintaining clinical response. In some embodiments, treating is inducing and maintaining clinical response. In some embodiments, treating is inducing clinical response. In some embodiments, treating is maintaining clinical response. In some embodiments, treating is inducing endoscopic improvement. In some embodiments, treating is maintaining endoscopic improvement. In some embodiments, treating is achieve endoscopic improvement. In some embodiments, treating is improving endoscopic remission. In some embodiments, treating is maintaining endoscopic remission. In some embodiments, treating is inducing histologic healing. In some embodiments, treating is maintaining histologic healing. In some embodiments, treating is improving stool frequency. In some embodiments, treating is maintaining improvement in stool frequency. In some embodiments, treating is improving endoscopic appearance of the mucosa. In some embodiments, treating is maintaining endoscopic improvement of the mucosa. In some embodiments, treating is improving endoscopic appearance of the mucosa during induction. In some embodiments, treating eliminates the need for corticosteroid use. In some embodiments, treating allows for reduced corticosteroid use. In some embodiments, treating allows for the use of a lower dose of a corticosteroid. In some embodiments, treating is achieving corticosteroid-free remission. In some embodiments, treating is sustaining corticosteroid-free remission. In some embodiments, treating is improving rectal bleeding. In some embodiments, treating is maintaining improvement in rectal bleeding. In some embodiments, treating is improving endoscopic subscore. In some embodiments, treating is maintaining improvement in endoscopic subscore.

In some embodiments, ulcerative colitis has been diagnosed using a 2-component Mayo Clinic Score. For example, in some embodiments, ulcerative colitis has been diagnosed using a score ranging from 0 to 9 for rectal bleeding and endoscopic findings. In some embodiments, ulcerative colitis has been diagnosed using a 3-component Mayo Clinic Score. For example, in some embodiments, ulcerative colitis has been diagnosed using a score ranging from 0 to 9 for stool frequency, rectal bleeding, and endoscopic findings. In some embodiments, ulcerative colitis has been diagnosed using a Total Mayo Score. For example, in some embodiments, ulcerative colitis has been diagnosed using a score ranging from 0 to 12 for stool frequency, rectal bleeding, endoscopic findings, and Physicians Global Assessment.

In some embodiments, improvement in ulcerative colitis is measured using a 2-component Mayo Clinic Score. In some embodiments, improvement in ulcerative colitis is measured using a 3-component Mayo Clinic Score. In some embodiments, improvement in ulcerative colitis is measured using a Total Mayo Score. In some embodiments, improvement in ulcerative colitis is measured by clinical remission. In some embodiments, improvement in ulcerative colitis is measured by lymphocyte reduction. In some embodiments, improvement in ulcerative colitis is measured by endoscopic improvement. In some embodiments, improvement in ulcerative colitis is measured by 6-point Mayo Score. For example, in some embodiments, improvement in ulcerative colitis is measured by stool frequency and rectal bleeding. In some embodiments, improvement in ulcerative colitis is statistically significant.

In some embodiments, Compound 1 is not recommended in an individual with active, severe infection. In some embodiments, Compound 1 is not recommended in an individual with an active infection. In some embodiments, Compound 1 is not recommended in an individual with a severe infection. In some embodiments, Compound 1 is not recommended in an individual with an active, severe infection until the infection is controlled. In some embodiments, Compound 1 is not recommended in an individual with an active infection until the infection is controlled. In some embodiments, Compound 1 is not recommended in an individual with a severe infection until the infection is controlled. In some embodiments, administration of Compound 1 is not started during an active infection. In some embodiments, an individual is monitored for infection. In some embodiments, administration of Compound 1 is stopped if an individual develops an infection. In some embodiments, administration of Compound 1 is stopped if infection becomes serious. In some embodiments, administration of Compound 1 is discontinued if an individual develops an infection. In some embodiments, Compound 1 is not administered to an individual with an infection. In some embodiments, Compound 1 is not administered during an active infection. In some embodiments, administration of Compound 1 is not started during active infection; an individual is monitored if an infection develops during administration; and administration is stopped if the infection becomes serious. In some embodiments, an infection is mild. In some embodiments, an infection is moderate. In some embodiments, an infection is severe. In some embodiments, an infection is serious. In some embodiments, an infection is a serious adverse event. In some embodiments, an infection is a respiratory infection.

In some embodiments, Compound 1 is administered without causing a severe adverse event. In some embodiments, Compound 1 is administered without causing a severe adverse event related to heart rate. In some embodiments, Compound 1 is administered without causing a severe adverse event related to heart rate change. In some embodiments, Compound 1 is administered without causing a severe adverse event related to elevated heart rate. In some embodiments, Compound 1 is administered without causing a severe adverse event related to bradycardia. In some embodiments, Compound 1 is administered without causing a severe adverse event related to AV block. In some embodiments, Compound 1 is administered without causing a severe adverse event related to AV conduction. In some embodiments, Compound 1 is administered without causing bradycardia. In some embodiments, Compound 1 is administered without causing AV block. In some embodiments, Compound 1 is administered without causing more than mild decrease in heart rate on first day of treatment (for example, >10 bpm). In some embodiments, Compound 1 is administered without a first-dose effect seen with other S1P receptor modulators. In some embodiments, Compound 1 is administered without a first-dose cardiovascular effect seen with other S1P receptor modulators. In some embodiments, Compound 1 is administered without symptomatic changes in heart rate. In some embodiments, Compound 1 is administered without symptomatic changes in heart rhythm. In some embodiments, Compound 1 is administered without requiring titration to avoid first-dose effect seen with other S1P receptor modulators.

In some embodiments, Compound 1 is administered without increasing a liver function test (LFT). In some embodiments, Compound 1 is administered without causing an elevated LFT. In some embodiments, Compound 1 is administered without increasing ALT. In some embodiments, Compound 1 is administered without increasing AST. In some embodiments, Compound 1 is administered without increasing ALT >3X ULN. In some embodiments, Compound 1 is administered without increasing ALT >2.5X ULN. In some embodiments, Compound 1 is administered without increasing ALT >2X ULN. In some embodiments, Compound 1 is administered without increasing ALT >1.5X ULN. In some embodiments, Compound 1 is administered without increasing AST >3X ULN. In some embodiments, Compound 1 is administered without increasing AST >2.5X ULN. In some embodiments, Compound 1 is administered without increasing AST >2X ULN. In some embodiments, Compound 1 is administered without increasing AST >1.5X ULN. In some embodiments, Compound 1 is administered without increasing bilirubin. In some embodiments, Compound 1 is administered without increasing bilirubin >3X ULN. In some embodiments, Compound 1 is administered without increasing bilirubin >2.5X ULN. In some embodiments, Compound 1 is administered without increasing bilirubin >2X ULN. In some embodiments, Compound 1 is administered without increasing bilirubin >1.5X ULN. In some embodiments, Compound 1 is administered without increasing gamma-glutamyl transferase (GGT). In some embodiments, Compound 1 is administered without increasing GGT >3X ULN. In some embodiments, Compound 1 is administered without increasing GGT >2.5X ULN. In some embodiments, Compound 1 is administered without increasing GGT >2X ULN. In some embodiments, Compound 1 is administered without increasing GGT >1.5X ULN.

In some embodiments, Compound 1 is administered without causing an abnormality in a pulmonary function test. In some embodiments, Compound 1 is administered without causing macular edema.

In some embodiments, the individual has had an inadequate response with, lost response to, been intolerant to, or demonstrated dependence on another agent for the treatment of an inflammatory bowel disease. In some embodiments, the individual has had an inadequate response with the other agent for the treatment of an inflammatory bowel disease. In some embodiments, the individual has lost response to another agent for the treatment of an inflammatory bowel disease. In some embodiments, the individual was intolerant to another agent for the treatment of an inflammatory bowel disease. In some embodiments, the individual requires continuous steroid therapy. In some embodiments, the other agent is at least one agent selected from: a tumor necrosis tumor necrosis factor (TNF) antagonist, a corticosteroid, an integrin antagonist, and immunosuppressive agent, and an aminosalicylate.

In some embodiments, the individual has had an inadequate response with, lost response to, or been intolerant to a conventional therapy. In some embodiments, the individual has had an inadequate response to conventional therapy. In some embodiments, the individual has lost response to conventional therapy. In some embodiments, the individual has been intolerant to conventional therapy. In some embodiments, the conventional therapy is selected from: at least one agent selected from: a tumor necrosis tumor necrosis factor (TNF) antagonist, a corticosteroid, an integrin antagonist, and immunosuppressive agent, and an aminosalicylate.

In some embodiments, the individual was previously administered a corticosteroid and/or an aminosalicylate. In some embodiments, the individual was previously administered a tumor necrosis tumor necrosis factor (TNF) antagonist, an integrin antagonist, and/or an immunosuppressive agent.

In some embodiments, the corticosteroid is an oral corticosteroid. In some embodiments, the TNF antagonist is a TNF-α blocker. In some embodiments, the aminosalicylate is a 5-aminosalicylate. In some embodiments, the integrin antagonist is referred to as an integrin receptor antagonist. In some embodiments, the TNF antagonist is referred to as a TNF blocker. In some embodiments, the immunosuppressive agent is referred to as an immunomodulator. In some embodiments, the prior conventional therapy is referred to as prior treatment.

S1P receptor agonists having agonist activity on the S1P₁ receptor have been shown to rapidly and reversibly induce lymphopenia (also referred to as peripheral lymphocyte lowering (PLL); Hale et al., Bioorg. Med. Chem. Lett., 14:3351-3355, 2004). This is attended by clinically useful immunosuppression by virtue of sequestering T- and B-cells in secondary lymphoid tissue (lymph nodes and Peyer's patches) and thus apart from sites of inflammation and organ grafts (Rosen et al., Immunol. Rev., 195:160-177, 2003; Schwab et al., Nature Immunol., 8:1295-1301, 2007). This lymphocyte sequestration, for example in lymph nodes, is thought to be a consequence of concurrent agonist-driven functional antagonism of the S1P₁ receptor on T-cells (whereby the ability of S1P to mobilize T-cell egress from lymph nodes is reduced) and persistent agonism of the S1P₁ receptor on lymph node endothelium (such that barrier function opposing transmigration of lymphocytes is increased) (Matloubian et al., Nature, 427:355-360, 2004; Baumruker et al., Expert Opin. Investig. Drugs, 16:283-289, 2007). It has been reported that agonism of the S1P₁ receptor alone is sufficient to achieve lymphocyte sequestration (Sanna et al., J. Biol. Chem., 279:13839-13848, 2004) and that this occurs without impairment of immune responses to systemic infection (Brinkmann et al., Transplantation, 72:764-769, 2001; Brinkmann et al., Transplant. Proc., 33:530-531, 2001).

That agonism of endothelial S1P₁ receptors has a broader role in promoting vascular integrity is supported by work implicating the S1P₁ receptor in capillary integrity in mouse skin and lung (Sanna et al., Nat. Chem. Biol., 2:434-441, 2006). Vascular integrity can be compromised by inflammatory processes, for example as may derive from sepsis, major trauma and surgery so as to lead to acute lung injury or respiratory distress syndrome (Johan Groeneveld, Vascul. Pharmacol., 39:247-256, 2003).

In one embodiment, the present invention encompasses compounds which are agonists of the S1P₁ receptor having selectivity over the S1P₃ receptor. Using a combined chemical approach with S1P receptor null mice, Sanna *et al.* reported that sustained bradycardia was induced by nonselective S1P receptor immunosuppressive agonists in wild-type mice but was abolished in S1P₃-/- mice whereas an S1P₁-selective agonist did not produce bradycardia. Thus suggesting that the S1P₃ receptor, and not the S1P₁ receptor, was responsible for bradycardia (Sanna et al., J. Biol. Chem., 279:13839-13848, 2004). Therefore, an S1P₁ receptor agonist selective over at least the S1P₃ receptor has advantages over current therapies by virtue of an enhanced therapeutic window, allowing better tolerability with higher dosing and thus improving efficacy as therapy. The present invention encompasses Compound 1 (and pharmaceutically acceptable salts, hydrates, and solvates thereof) which is an agonist of the S1P₁ receptor and has exhibited no or substantially no bradycardia in male Sprague-Dawley^{®} rats (see WO2010/011316, Example 9).

A phase 1 study with Compound 1 was conducted with single dosing at 0.1 mg, 0.35 mg, 1 mg, 3 mg, and 5 mg. Compound 1 was administered as the L-arginine salt. Lower doses of 0.1 mg through 3 mg were well tolerated by subjects with only minor adverse events reported, the most common of which were headache and contact dermatitis. A dose-dependent reduction in heart rate was seen in all doses >0.35 mg, however, no adverse events related to bradycardia were reported at doses lower than the 5 mg dose. Dose limiting adverse events were observed at the dose of 5 mg, with 3 (50%) subjects experiencing 4 AEs of bradycardia with first or second degree atrioventricular (AV) block, which resulted in discontinuation of dose escalation. The maximum tolerated dose in the study was 3 mg. There were no deaths or serious adverse events in the study.

There were no other clinically significant safety issues with respect to vital signs, ECGs, pulmonary function tests, ophthalmoscopy, or clinical laboratory tests with the exception of expected pharmacological effects on peripheral blood lymphocyte counts. Dosing at the 3 and 5 mg induced a dose responsive decline in the absolute number of peripheral blood B cells, T cells, NK cells, and all T cell subsets except TEM cells. Total peripheral blood lymphocyte (PBL) counts were reduced by 2-4 hours after dosing, reaching a nadir by hour 8 which persisted for 24 hours with recovery to baseline over the next 4 days. PBL counts were reduced by ~40% and ~55% at the 3 mg and 5 mg dose levels. TEM cells do not express CCR7 and are able to recirculate independently of S1P receptor expression. These findings are therefore consistent with the anticipated pharmacodynamic effects of S1P receptor agonists in preclinical studies and in humans (Gergely et al., Br J Pharmacol 167(5):1035-1047, 2012; Brossard et al., Br J Clin Pharmacol 2013 Apr 18. doi:10.1111/bcp.12129. [Epub ahead of print] PubMed PMID: 23594176, and Kovarik et al., J Clin Pharmacol 44(5):532-537, 2004.)

S1P₁ receptor agonists are useful to treat or prevent conditions where suppression of the immune system or agonism of the S1P₁ receptor is in order, such as diseases and disorders mediated by lymphocytes, transplant rejection, autoimmune diseases and disorders, inflammatory diseases and disorders, and conditions that have an underlying defect in vascular integrity or that relate to angiogenesis such as may be pathologic.

S1P₁ receptor agonists are useful for treating or preventing conditions where suppression of the immune system or agonism of the S1P₁ receptor is in order, such as diseases and disorders mediated by lymphocytes, transplant rejection, autoimmune diseases and disorders, inflammatory diseases and disorders (*e.g.,* acute and chronic inflammatory conditions), cancer, and conditions that have an underlying defect in vascular integrity or that are associated with angiogenesis such as may be pathologic *(e.g.,* as may occur in inflammation, tumor development and atherosclerosis). Such conditions where suppression of the immune system or agonism of the S1P₁ receptor is in order include diseases and disorders mediated by lymphocytes; conditions that have an underlying defect in vascular integrity; autoimmune diseases and disorders; inflammatory diseases and disorders (e.g., acute and chronic inflammatory conditions); acute or chronic rejection of cells; tissue or solid organ grafts; arthritis, including psoriatic arthritis, and rheumatoid arthritis; diabetes, including type I diabetes; demyelinating disease, including multiple sclerosis; ischemia-reperfusion injury, including renal and cardiac ischemia-reperfusion injury; inflammatory skin disease, including psoriasis, atopic dermatitis, and acne; hyperproliferative skin disease, including acne; inflammatory bowel disease, including Crohn's disease, and ulcerative colitis; systemic lupus erythematosus; asthma; uveitis; myocarditis; allergy; atherosclerosis; brain inflammation, including Alzheimer's disease, and brain inflammatory reaction following traumatic brain injury; ankylosing spondylitis; central nervous system disease, including spinal cord injury, or cerebral infarction; pathologic angiogenesis, including as may occur in primary and metastatic tumor growth; rheumatoid arthritis; diabetic retinopathy, atherosclerosis; cancer; chronic pulmonary disease; acute lung injury; acute respiratory disease syndrome; sepsis; and the like. In addition, S1P₁ receptor agonists are useful for treating microbial infections, and viral infections or diseases.

The S1P₁ receptor-associated disorder treated according to the claimed invention is ulcerative colitis. In some embodiments, the S1P₁ receptor-associated disorder is moderately to severely active ulcerative colitis. In some embodiments, the S1P₁ receptor-associated disorder is moderately active ulcerative colitis. In some embodiments, the S1P₁ receptor-associated disorder is severely active ulcerative colitis. In some embodiments, the S1P₁ receptor-associated disorder is mildly to moderately active ulcerative colitis. In some embodiments, the S1P₁ receptor-associated disorder is mildly active ulcerative colitis.

According to the claimed invention, Compound 1, or a pharmaceutically acceptable salt, hydrate, or solvate thereof, is administered in an amount equivalent to 2 mg of Compound 1.

According to the claimed invention, Compound 1, or a pharmaceutically acceptable salt, hydrate, or solvate thereof is administered once daily to the individual.

According to the claimed invention, the Compound 1, or a pharmaceutically acceptable salt, hydrate, or solvate thereof, is administered orally.

In some embodiments, the Compound 1, or a pharmaceutically acceptable salt, hydrate, or solvate thereof, is formulated as a capsule or tablet suitable for oral administration.

In some embodiments, the Compound 1, or a pharmaceutically acceptable salt, hydrate, or solvate thereof, is selected from: Compound 1; a calcium salt of Compound 1; and an L-arginine salt of Compound 1. In some embodiments, the Compound 1, or a pharmaceutically acceptable salt, hydrate, or solvate thereof, is an L-arginine salt of Compound 1. In some embodiments, the Compound 1, or a pharmaceutically acceptable salt, hydrate, or solvate thereof, is an anhydrous, non-solvated crystalline form of an L-arginine salt of Compound 1. In some embodiments, the Compound 1, or a pharmaceutically acceptable salt, hydrate, or solvate thereof, is an anhydrous, non-solvated crystalline form of Compound 1.

In some embodiments, the individual also is administered a therapeutic dose of an oral 5-ASA compound.

In some embodiments, the individual also is administered a therapeutic dose of an oral corticosteroid therapy. In some embodiments, the corticosteroid is prednisone, e.g., prednisone at a dose ≤ 20 mg/day, or an equivalent steroid. In some embodiments, the corticosteroid is budesonide, e.g., at a dose ≤ 9 mg/day, or an equivalent steroid.

In some embodiments, the individual also is administered a therapeutic dose of an immunosuppressive agent. In some embodiments, the individual also is administered a therapeutic dose of azathioprine. In some embodiments, the individual also is administered a therapeutic dose of 6-mercaptopurine.

In some embodiments, the individual also is administered a therapeutic dose of a probiotic. In some embodiments, the individual also is administered a therapeutic dose of Culturelle. In some embodiments, the individual also is administered a therapeutic dose of *Saccharomyces boulardii.*

In some embodiments, the individual also is administered a therapeutic dose of an antidiarrheal. In some embodiments, the individual also is administered a therapeutic dose of loperamide. In some embodiments, the individual also is administered a therapeutic dose of diphenoxylate with atropine.

Some embodiments of the present invention include a method of producing a pharmaceutical composition for "combination-therapy" comprising admixing at least one compound according to any of the compound embodiments disclosed herein, together with at least one known pharmaceutical agent as described herein and a pharmaceutically acceptable carrier.

Also provided are pharmaceutical compositions comprising Compound 1, or, a pharmaceutically acceptable salt, a hydrate or solvate thereof and, optionally, one or more pharmaceutically acceptable carriers. Also provided are pharmaceutical compositions comprising Compound 1, or, a pharmaceutically acceptable salt, a hydrate or solvate thereof, optionally, one or more pharmaceutically acceptable carriers. The carrier(s) must be "acceptable" in the sense of being compatible with the other ingredients of the formulation and not overly deleterious to the recipient thereof.

In some embodiments, Compound 1, or, a pharmaceutically acceptable salt, a hydrate or solvate thereof, is administered as a raw or pure chemical, for example as a powder in capsule formulation.

In some embodiments, Compound 1, or, a pharmaceutically acceptable salt, a hydrate or solvate thereof, is formulated as a pharmaceutical composition further comprising one or more pharmaceutically acceptable carriers.

Pharmaceutical compositions may be prepared by any suitable method, typically by uniformly mixing the active compound(s) with liquids or finely divided solid carriers, or both, in the required proportions and then, if necessary, forming the resulting mixture into a desired shape.

Conventional excipients, such as binding agents, fillers, acceptable wetting agents, tabletting lubricants and disintegrants may be used in tablets and capsules for oral administration. The compounds described herein can be formulated into pharmaceutical compositions using techniques well known to those in the art. Suitable pharmaceutically acceptable carriers, outside those mentioned herein, are known in the art; for example, see Remington, The Science and Practice of Pharmacy, 20th Edition, 2000, Lippincott Williams & Wilkins, (Editors: Gennaro *et al*.)

For oral administration, the pharmaceutical composition may be in the form of, for example, a tablet or capsule. The pharmaceutical composition is preferably made in the form of a dosage unit containing a particular amount of the active ingredient. Examples of such dosage units are capsules, tablets, powders, granules or suspensions, with conventional additives such as lactose, mannitol, corn starch or potato starch; with binders such as crystalline cellulose, cellulose derivatives, acacia, corn starch or gelatins; with disintegrators such as corn starch, potato starch or sodium carboxymethyl-cellulose; and with lubricants such as talc or magnesium stearate. Solid form preparations include powders, tablets, pills, capsules, cachets, suppositories, and dispersible granules. A solid carrier can be one or more substances which may also act as diluents, flavoring agents, solubilizers, lubricants, suspending agents, binders, preservatives, tablet disintegrating agents, or encapsulating materials.

In powders, the carrier is a finely divided solid which is in a mixture with the finely divided active component.

In tablets, the active component is mixed with the carrier having the necessary binding capacity in suitable proportions and compacted to the desired shape and size.

The powders and tablets may contain varying percentage amounts of the active compound. A representative amount in a powder or tablet may be from 0.5 to about 90 percent of the active compound. However, an artisan would know when amounts outside of this range are necessary. Suitable carriers for powders and tablets include magnesium carbonate, magnesium stearate, talc, sugar, lactose, pectin, dextrin, starch, gelatin, tragacanth, methylcellulose, sodium carboxymethyl cellulose, a low melting wax, cocoa butter, and the like. The term "preparation" is includes the formulation of the active compound with encapsulating material as carrier providing a capsule in which the active component, with or without carriers, is surrounded by a carrier, which is thus in association with it. Similarly, cachets and lozenges are included. Tablets, powders, capsules, pills, cachets, and lozenges can be used as solid forms suitable for oral administration.

The pharmaceutical preparations are preferably in unit dosage forms. In such form, the preparation is subdivided into unit doses containing appropriate quantities of the active component. The unit dosage form can be a packaged preparation, the package containing discrete quantities of preparation, such as packeted tablets or capsules. Also, the unit dosage form can be a capsule or tablet itself, or it can be the appropriate number of any of these in packaged form.

### EXAMPLES

### EXAMPLE 1

Formulations composed of immediate-release, hard gelatin capsules containing an L-arginine salt of Compound 1 were prepared as shown in **Table 1.**

**TABLE 1**

| | **Formulation** | | | | |
|---|---|---|---|---|---|
| | **0.1 mg** | **0.35 mg** | **0.5 mg** | **1 mg** | **2 mg** |
| L-arginine salt of Compound 1 (mg/capsule) | 0.14 | 0.48 | 0.69 | 1.38 | 2.76 |
| Empty capsule weight (mg)* | 38.0 | 61.0 | 61.0 | 61.0 | 61.0 |
| Total capsule target weight (mg)** | 38.14 | 61.48 | 61.69 | 62.38 | 63.76 |

| | | | | | |
|---|---|---|---|---|---|
| *Approximate weight. Based on capsule specification **Theoretical total weight calculated by combining fill and empty capsule weights together | | | | | |

Placebo formulations composed of hard gelatin capsules containing microcrystalline cellulose was also prepared as shown in **Table 2.**

**TABLE 2**

| | **Placebo for 0.1 mg** | **Placebo for 0.35 mg and 1 mg** | **Placebo for 0.5 mg, 1 mg, and 2 mg** |
|---|---|---|---|
| Microcrystalline cellulose - Avicel PH102 (mg/capsule)* | 0.0 | 0.0 | 1.0^{*} |
| Empty capsule weight (mg) ** | 38.0 | 61.0 | 61.0 |
| Total capsule target weight (mg)*** | 38.0 | 61.0 | 62.0 |

| | | | |
|---|---|---|---|
| *Approximate weight ± 15% **Approximate weight. Based on capsule specification ***Theoretical total weight calculated by combining fill and empty capsule weights together | | | |

### EXAMPLE 2

A randomized, double-blind, placebo-controlled, sequential, ascending, multiple dose study to assess the safety, tolerability, and pharmacokinetics of the L-arginine salt of Compound 1 administered to healthy adult subjects was conducted. This study was designed to evaluate the safety, tolerability, pharmacokinetics, and pharmacodynamics of the L-arginine salt of Compound 1.

**Tables 3** and **4** below provide a summary of demographic data by treatment group and a more detailed analysis of the safety population.

**TABLE 3**

| | Placebo | 0.7 mg | 1.35 mg | 2.0 mg | 0.35 mg, 2.0 mg | 0.5 mg, 3.0 mg |
|---|---|---|---|---|---|---|
| No. of Subjects Randomized | 10 | 10 | 10 | 10 | 10 | 10 |
| No. (%) of Subjects in Safety Population^{a} | 10 (100.0%) | 10 (100.0%) | 10 (100.0%) | 10 (100.0%) | 10 (100.0%) | 10 (100.0%) |
| No. (%) of Subjects Who Completed Study^{a} | 10 (100.0%) | 10 (100.0%) | 9 (90.0%) | 10 (100.0%) | 10 (100.0%) | 10 (100.0%) |
| No. (%) of Subjects Withdrawn Early from Study^{a} | 0 (0.0%) | 0 (0.0%) | 1 (10.0%) | 0 (0.0%) | 0 (0.0%) | 0 (0.0%) |
| Withdrawal of Informed Consent | 0 | 0 | 1 (100.0%) | 0 | 0 | 0 |

| | | | | | | |
|---|---|---|---|---|---|---|
| ^{a} Number of group subjects in each column is used as the denominator for percentage calculations. | | | | | | |

**TABLE 4**

| Demographics | Placebo (N=10) | 0.7 mg (N=10) | 1.35 mg (N=10) | 2.0 mg (N=10) | 0.35 mg, 2.0 mg (N=10) | 0.5 mg, 3.0 mg (N=10) |
|---|---|---|---|---|---|---|
| Age (years) | | | | | | |
| N | 10 | 10 | 10 | 10 | 10 | 10 |
| Mean (SD) | 35.6 (7.4) | 34.2 (8.8) | 31.4 (9.0) | 30.1 (7.0) | 32.8 (6.0) | 29.0 (7.2) |
| Median | 36.0 | 35.0 | 29.5 | 26.5 | 33.5 | 27.0 |
| Min - Max | 21-45 | 18 - 44 | 19-45 | 21-44 | 22-41 | 20-41 |
| CV | 20.8% | 25.6% | 28.7% | 23.2% | 18.2% | 24.7% |

| Age Group | | | | | | |
|---|---|---|---|---|---|---|
| 18-24 | 1 (10.0%) | 2 (20.0%) | 2 (20.0%) | 1 (10.0%) | 1 (10.0%) | 3 (30.0%) |
| 25-34 | 3 (30.0%) | 2 (20.0%) | 5 (50.0%) | 6 (60.0%) | 5 (50.0%) | 4 (40.0%) |
| 35-45 | 6 (60.0%) | 6 (60.0%) | 3 (30.0%) | 3 (30.0%) | 4 (40.0%) | 3 (30.0%) |

| Race | | | | | | |
|---|---|---|---|---|---|---|
| White | 8 (80.0%) | 7 (70.0%) | 8 (80.0%) | 7 (70.0%) | 6 (60.0%) | 3 (30.0%) |
| Black or African American | 2 (20.0%) | 3 (30.0%) | 1 (10.0%) | 3 (30.0%) | 4 (40.0%) | 5 (50.0%) |
| Asian | 0 (0.0%) | 0 (0.0%) | 1 (10.0%) | 0 (0.0%) | 0 (0.0%) | 0 (0.0%) |
| American Indian or Alaska Native | 0 (0.0%) | 0 (0.0%) | 0 (0.0%) | 0 (0.0%) | 0 (0.0%) | 2 (20.0%) |

| Ethnicity | | | | | | |
|---|---|---|---|---|---|---|
| Hispanic Or Latino | 6 (60.0%) | 3 (30.0%) | 5 (50.0%) | 5 (50.0%) | 3 (30.0%) | 5 (50.0%) |
| Not Hispanic Or Latino | 4 (40.0%) | 7 (70.0%) | 5 (50.0%) | 5 (50.0%) | 7 (70.0%) | 5 (50.0%) |

| Sex | | | | | | |
|---|---|---|---|---|---|---|
| Male | 2 (20.0%) | 5 (50.0%) | 3 (30.0%) | 4 (40.0%) | 5 (50.0%) | 4 (40.0%) |
| Female | 8 (80.0%) | 5 (50.0%) | 7 (70.0%) | 6 (60.0%) | 5 (50.0%) | 6 (60.0%) |

| Weight (kg) | | | | | | |
|---|---|---|---|---|---|---|
| N | 10 | 10 | 10 | 10 | 10 | 10 |
| Mean (SD) | 75.7 (8.7) | 79.6 (14.2) | 73.9 (12.9) | 74.4 (17.3) | 78.2 (12.1) | 79.1 (9.4) |
| Median | 76.3 | 82.7 | 73.2 | 73.1 | 76.2 | 79.8 |
| Min - Max | 64.3 - 88.7 | 54.5 - 97.8 | 54.7 - 95.3 | 51.6 - 98.1 | 62.4 - 96.9 | 67.0 - 98.4 |
| CV | 11.5% | 17.9% | 17.5% | 23.2% | 15.5% | 11.9% |

| Height (cm) | | | | | | |
|---|---|---|---|---|---|---|
| N | 10 | 10 | 10 | 10 | 10 | 10 |
| Mean (SD) | 160.6 (10.6) | 166.0 (8.4) | 165.4 (9.0) | 166.0 (12.2) | 169.2 (9.0) | 169.0 (6.4) |
| Median | 161.0 | 165.3 | 162.0 | 165.8 | 168.3 | 168.5 |
| Min - Max | 145.0 - 180.5 | 156.0 - 179.0 | 157.0 - 183.0 | 150.0 - 186.5 | 156.0 - 185.0 | 154.5 - 176.5 |
| CV | 6.6% | 5.0% | 5.5% | 7.3% | 5.3% | 3.8% |

| BMI (kg/m²) | | | | | | |
|---|---|---|---|---|---|---|
| N | 10 | 10 | 10 | 10 | 10 | 10 |
| Mean (SD) | 29.5 (3.8) | 28.9 (4.5) | 26.9 (3.1) | 27.0 (5.8) | 27.2 (2.1) | 27.7 (2.2) |
| Median | 29.9 | 29.8 | 27.3 | 26.0 | 27.4 | 27.9 |
| Min - Max | 23.8 - 34.1 | 21.5 - 33.8 | 21.8 - 30.8 | 19.1 - 40.1 | 24.1 - 30.5 | 23.5 - 31.6 |
| CV | 12.7% | 15.6% | 11.4% | 21.6% | 7.7% | 8.0% |

Cohort 1 was dosed with 0.7 mg (by administering two 0.35 mg formulations) for 21 days. Cohort 2 was dosed with 1.35 mg (by administering both a 0.35 mg formulation and a 1 mg formulation) for 21 days. Cohort 3 was dosed with 2.0 mg for 21 days. Cohort 4 was dosed with 0.35 mg for 7 days and then with 2.0 mg for 14 days. Cohort 5 was dosed with 0.5 for 7 days and then with 3.0 mg (by administering both a 1 mg formulation and a 2 mg formulation) for 14 days.

The following safety assessments were conducted: physical examinations with ophthalmoscopy, clinical laboratory tests (serum chemistry, coagulation, and urinalysis), vital signs, continuous telemetry (12 lead ECG), safety ECGs, pulmonary function testing (PFT), serum protein electrophoresis (SPEP) and serum immunoelectrophoresis (IEP), and adverse event reporting.

The L-arginine salt of Compound 1 was tolerated at all dose levels. The most common adverse events included contact dermatitis and leukopenia, followed by constipation, diarrhea, nausea, and abdominal pain. The contact dermatitis observed is consistent with what is generally seen with the adhesive tape from the ECG leads used in the study and did not occur more frequently in the treated group. The majority of adverse events were mild. There were no other clinically significant safety issues with respect to vital signs, ECGs, PFTs, ophthalmoscopy, or clinical laboratory tests. No subjects were discontinued due to an adverse event. No SAEs or deaths occurred during the study.

No second degree heart block was found. Three subjects developed new (not present before dosing) 1^{st} degree atrioventricular block: 1 subject in placebo group, 1 subject in 2mg group, and 1 subject in 0.5, 3mg group. One subject (1.35mg dose) had mildly abnormal reproducible (NCS) pulmonary function test findings (FEV1 below 80%, FVC) post-dose.

Two subjects had mildly abnormal non-clinically significant post-treatment liver function tests (elevated alanine aminotransferase (ALT) & aspartate aminotransferase (AST) > 2X upper limit of normal (ULN)): 1 subject in 2 mg group and 1 subject in 0.5 escalating to 3 mg group.

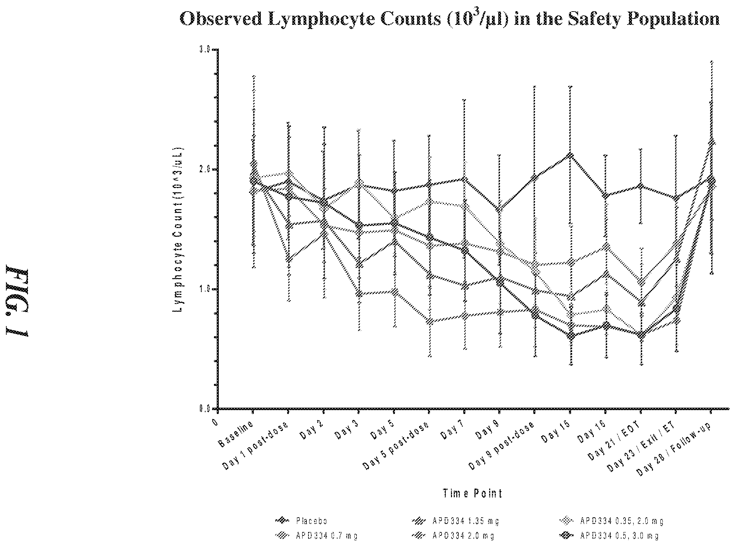

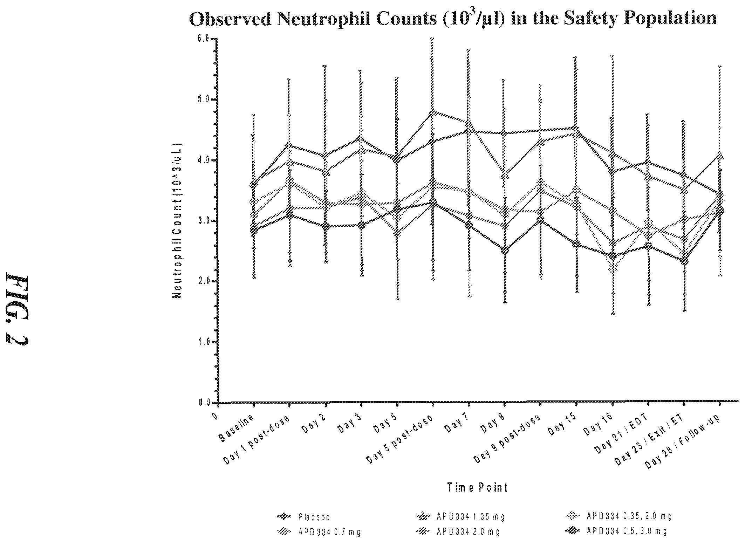

No clinically significant change from baseline in ophthalmoscopy findings were found on exam. **Figure 1** shows observed lymphocyte counts (10³/µl) in the safety population. **Figure 2** shows observed neutrophil counts (10³/µl) in the safety population. **Table 5** shows a summary of percent change from baseline at day 21 in lymphocytes (10³/µl): safety population.

**TABLE 5**

| **Parameter** | | **Baseline** | **On Treatment** | **Percent Change from Baseline** | | |
|---|---|---|---|---|---|---|
| **Treatment** | **N** | **Mean (SD)** | **Mean (SD)** | **Mean (SE)** | **Median** | **Min, Max** |
| Placebo | 10 | 1.81 (0.44) | 1.86 (0.31) | 5.08 (4.24) | 7.67 | -17.39 to 23.08 |
| 0.7 mg | 10 | 1.82 (0.46) | 1.06 (0.28) | -41.03 (3.19) | -40.37 | -55.56 to -23.08 |
| 1.35 mg | 9 | 2.03 (0.83) | 0.89 (0.32) | -53.43 (4.74) | -51.85 | -68.75 to -30.77 |
| 2.0 mg | 10 | 2.05 (0.61) | 0.62 (0.18) | -68.81 (2.60) | -69.44 | -80.00 to -55.56 |
| 0.35, 2.0 mg | 10 | 1.93 (0.45) | 0.62 (0.18) | -67.34 (2.13) | -67.54 | -77.27 to -57.14 |
| 0.5, 3.0 mg | 10 | 1.90 (0.60) | 0.62 (0.25) | -66.16 (3.40) | -65.02 | -85.19 to -53.85 |
| Note: Baseline was the last measure prior to first dose. | | | | | | |

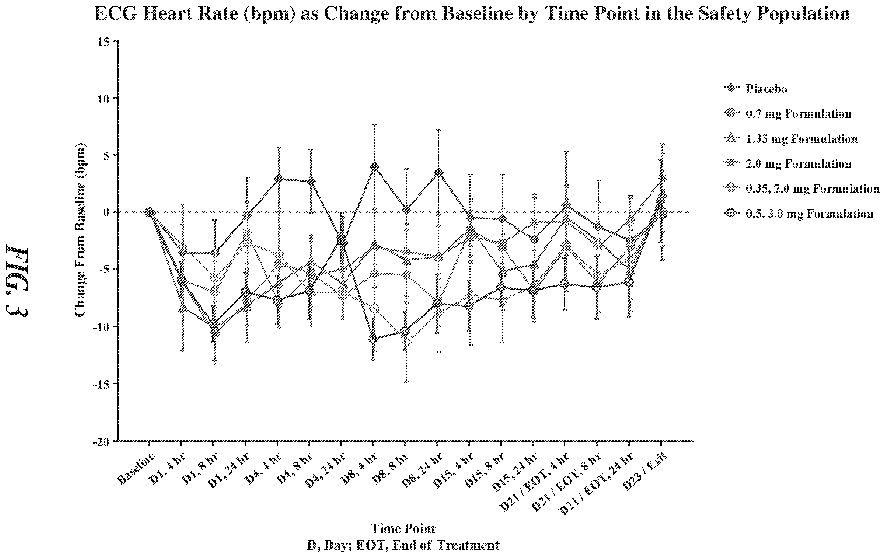

**Table 6** shows a summary of change from baseline in minimum value for post-dose (day 1 to day 28) in heart rate (BPM): safety population. See, also **Figure 3****.**

**TABLE 6**

| **Parameter** | | **Baseline** | **On Treatment** | **Change from Baseline** | | |
|---|---|---|---|---|---|---|
| **Treatment** | **N** | **Mean (SD)** | **Mean (SD)** | **Mean (SE)** | **Median** | **Min, Max** |
| **Minimum Value for Post-dose (Day 1 to Day 28) in Heart Rate (BPM)** | | | | | | |
| Placebo | 10 | 61.20 (9.73) | 56.50 (5.99) | -4.70 (2.17) | -2.00 | -17.00 to 4.00 |
| 0.7 mg | 10 | 59.70 (3.89) | 54.20 (4.05) | -5.50 (0.95) | -5.00 | -12.00 to -1.00 |
| 1.35 mg | 10 | 60.60 (6.62) | 54.40 (4.09) | -6.20 (1.91) | -5.00 | -18.00 to 3.00 |
| 2.0 mg | 10 | 59.30 (4.72) | 51.00 (4.78) | -8.30 (1.40) | -7.50 | -16.00 to -3.00 |
| 0.35, 2.0 mg | 10 | 62.60 (12.14) | 52.00 (4.74) | -10.60 (3.51) | -7.00 | -35.00 to 3.00 |
| 0.5, 3.0 mg | 10 | 61.10 (3.93) | 53.80 (4.73) | -7.30 (1.33) | -6.50 | -14.00 to -2.00 |
| Note: Baseline was defined as minimum of pre-dose values. | | | | | | |

**Table 7** shows a summary of change from baseline in minimum value for post-dose (day 1 to day 28) in systolic BP (mmHg): safety population.

**TABLE 7**

| **Parameter** | | **Baseline** | **On Treatment** | **Change from Baseline** | | |
|---|---|---|---|---|---|---|
| **Treatment** | **N** | **Mean (SD)** | **Mean (SD)** | **Mean (SE)** | **Median** | **Min, Max** |
| **Minimum Value for Post-dose (Dav 1 to Dav 28) in Systolic BP (mmHg)** | | | | | | |
| Placebo | 10 | 104.90 (4.84) | 95.40 (6.19) | -9.50 (1.75) | -8.00 | -23.00 to -3.00 |
| 0.7 mg | 10 | 105.80 (8.24) | 96.10 (7.72) | -9.70 (1.57) | -9.50 | -16.00 to -1.00 |
| 1.35 mg | 10 | 109.00 (10.62) | 100.10 (8.99) | -8.90 (2.79) | -8.00 | -22.00 to 3.00 |
| 2.0 mg | 10 | 100.60 (8.18) | 90.80 (8.73) | -9.80 (1.50) | -9.00 | -18.00 to -4.00 |
| 0.35, 2.0 mg | 10 | 107.30 (8.60) | 94.30 (12.50) | -13.00 (3.03) | -13.00 | -28.00 to 0.00 |
| 0.5, 3.0 mg | 10 | 101.00 (12.00) | 93.20 (8.66) | -7.80 (2.36) | -5.00 | -18.00 to 5.00 |
| Note: Baseline was defined as minimum of pre-dose values. | | | | | | |

**Table 8** shows a summary of change from baseline in minimum value for post-dose (day 1 to day 28) in diastolic BP (mmHg): safety population.

**TABLE 8**

| **Parameter** | | **Baseline** | **On Treatment** | **Change from Baseline** | | |
|---|---|---|---|---|---|---|
| **Treatment** | **N** | **Mean (SD)** | **Mean (SD)** | **Mean (SE)** | **Median** | **Min, Max** |
| **Minimum Value for Post-dose (Day 1 to Dev 28) in Diastolic BP (mmHg)** | | | | | | |
| Placebo | 10 | 58.50 (4.97) | 52.20 (4.71) | -6.30 (1.44) | -5.50 | -13.00 to 1.00 |
| 0.7 mg | 10 | 62.10 (8.84) | 56.00 (8.31) | -6.10 (1.92) | -6.00 | -16.00 to 2.00 |
| 1.35 mg | 10 | 58.90 (7.96) | 53.70 (4.03) | -5.20 (1.93) | -7.50 | -11.00 to 6.00 |
| 2.0 mg | 10 | 56.50 (5.62) | 45.30 (7.42) | -11.20 (1.88) | -9.00 | -21.00 to -4.00 |
| 0.35, 2.0 mg | 10 | 58.70 (6.48) | 50.80 (5.65) | -7.90 (2.00) | -6.00 | -19.00 to -1.00 |
| 0.5, 3.0 mg | 10 | 56.50 (6.77) | 50.20 (4.21) | -6.30 (1.89) | -7.00 | -13.00 to 3.00 |
| Note: Baseline was defined as minimum of pre-dose values. | | | | | | |

**Table 9** shows a summary of change from baseline in maximum value for post-dose (day 1 to day 23) in QTc (MS): safety population.

**TABLE 9**

| **Parameter** | | **Baseline** | **On Treatment** | **Change from Baseline** | | |
|---|---|---|---|---|---|---|
| **Treatment** | **N** | **Mean (SD)** | **Mean (SD)** | **Mean (SE)** | **Median** | **Min, Max** |
| **Maximum Value for Post-dose (Day 1 to Day 23) in QTc (MS)** | | | | | | |
| Placebo | 10 | 416.70 (10.40) | 416.10 (14.79) | -0.60 (3.39) | -2.50 | -11.00 to 24.00 |
| 0.7 mg | 10 | 414.80 (15.90) | 418.40 (16.56) | 3.60 (2.20) | 3.50 | -8.00 to 16.00 |
| 1.35 mg | 10 | 415.40 (14.65) | 423.60 (16.14) | 8.20 (3.10) | 6.00 | -5.00 to 25.00 |
| 2.0 mg | 10 | 417.20 (6.51) | 421.20 (10.61) | 4.00 (2.53) | 4.50 | -8.00 to 13.00 |
| 0.35, 2.0 mg | 10 | 411.20 (19.10) | 411.10 (19.88) | -0.10 (3.24) | 0.50 | -19.00 to 17.00 |
| 0.5, 3.0 mg | 10 | 419.50 (15.34) | 425.10 (13.90) | 5.60 (2.18) | 5.00 | -5.00 to 15.00 |
| Note: Baseline was defined as maximum of pre-dose values. | | | | | | |

In conclusion, the clinical trial showed a dose-dependent effect on lymphocyte lowering with maximal effect at 2 mg dose. The L-arginine salt of Compound 1 was well tolerated at all doses tested.

### EXAMPLE 3

A randomized, double-blind, placebo-controlled, parallel-group, dose-ranging study to assess safety and efficacy of two orally administered doses (1 mg and 2 mg) of etrasimod in patients with ulcerative colitis was conducted. **Table 10** provides a summary of demographic data by treatment group.

**TABLE 10**

| | | **Placebo (n=54)** | **L-arginine salt of Compound 1 1 mg (n=52)** | **L-arginine salt of Compound 1 2 mg (n=50)** |
|---|---|---|---|---|
| Mean Age, Years | | 44.8 ± 14.9 | 43.2 ± 12.2 | 40.4 ± 12.4 |
| Sex, n (%) | Male | 32 (59.3) | 30 (57.7) | 27 (54.0) |
| | Female | 22 (40.7) | 22 (42.3) | 23 (46.0) |
| Race, n (%) | White | 51 (94.4) | 48 (92.3) | 49 (98.0) |
| | Non-white | 3 (5.6) | 4 (7.7) | 1 (2.0) |
| Weight, kg | | 75.8 ± 16.2 | 73.7 ± 13.4 | 70.4 ± 16.7 |
| Duration of UC, years (mean ± SD) | | 8.6 ± 7.2 | 7.0 ± 6.1 | 6.2 ± 4.7 |
| Mean Total Mayo Clinic Score (Total MCS) | | 8.7 ± 1.7 | 8.8 ± 1.4 | 8.9 ± 1.5 |
| 3-Component Mayo Clinic Score (rectal bleeding, stool frequency, endoscopy) (Modified MCS) | | 6.5 ± 1.5 | 6.5 ± 1.2 | 6.6 ± 1.2 |
| Concomitant Medication Use, n (%) | Oral Corticosteroids | 16 (29.6) | 13 (25.0) | 18 (36.0) |
| Previous Medication Use, n (%) | Aminosalicylate | 53 (98.1) | 49 (94.2) | 46 (92.0) |
| | TNF-antagonist | 18 (33.3) | 15 (28.8) | 17 (34.0) |
| | Integrin antagonist | 12 (22.2) | 4 (7.7) | 7 (14.0) |
| | Immunosuppressive agent | 33 (61.1) | 17 (32.7) | 26 (52.0) |
| CRP, median (range), mg/L | | 3.63 (0.31-59.10) | 4.85 (0.21-245.00) | 4.92 (0.29-76.90) |
| FC, median (range), µg/g | | 1429 (67-16,226) | 1210 (30-24,190) | 1449 (71-21,559) |

**TABLE 11**

| | **Placebo** | **L-arginine salt of Compound 1** | **L-arginine salt of Compound 1** |
|---|---|---|---|
| Received Study Treatment | 54 | 52 | 50 |
| Completed, n (%) | 48 (88.9) | 47 (90.4) | 46 (92.0) |
| Early Discontinuation, n (%) | 6 (11.1) | 5 (9.6) | 4 (8.0) |

Patients were randomized into a double-blind, placebo-controlled study to receive once daily (q.d.) doses of the L-arginine salt of Compound 1 (1 mg or 2 mg) or matching placebo in a 1:1:1 ratio for 12 weeks. The trial enrolled 156 patients with moderate to severe ulcerative colitis (3-component Mayo score of 4-9 that includes endoscopic subscore ≥2, rectal bleeding score ≥1).

The treatment formulation was composed of immediate-release, hard gelatin capsules containing L-arginine salt of Compound 1. The placebo was composed of hard gelatin capsules containing microcrystalline cellulose.

The patients had demonstrated, over the previous 5 year period, an inadequate response to, loss of response to, or intolerance of at least one of the following agents:
Oral 5-aminosalicylates (5-ASAs) (e.g., mesalamines);
Corticosteroids wherein the patient exhibited signs and symptoms of persistently active disease despite a history of at least one 4-week induction regimen that included a dose equivalent to prednisone 30 mg daily; or 2 failed attempts to taper corticosteroids to below a dose equivalent to prednisone 10 mg daily; or a history of intolerance of corticosteroids (including, but not limited to Cushing's syndrome, osteopenia/osteoporosis, hyperglycemia, insomnia, and infection);
Immunosuppressives, wherein the patient exhibited signs and symptoms of persistently active disease despite a history of at least one 8-week regimen of oral azathioprine (≥ 1.5 mg/kg) or 6-mercaptopurine mg/kg (≥ 0.75 mg/kg); or a history of intolerance of at least one of these immunosuppressive (including, but not limited to nausea/vomiting, abdominal pain, pancreatitis, LFT abnormalities, lymphopenia, TPMT genetic mutation, infection);
TNFα antagonists, wherein the patient exhibited signs and symptoms of persistently active disease despite a history of completing an induction regimen with at least one of the following: infliximab, adalimumab, or golimumab at doses per the current labeling and/or institutional standard of care; or recurrence of symptoms during maintenance dosing with infliximab, adalimumab, or golimumab following prior clinical benefit (discontinuation despite clinical benefit does not qualify); or a history of intolerance to infliximab, adalimumab, or golimumab (including, but not limited to infusion- or injection- related reaction, demyelination, congestive heart failure, infection); or
Integrin antagonists, wherein the patient exhibited recurrence of symptoms during maintenance dosing with vedolizumab following prior clinical benefit (discontinuation despite clinical benefit does qualify); or a history of intolerance to vedolizumab (including, but not limited to infusion related reaction).

Patients were instructed to take their capsule on an empty stomach (overnight fast of approximately 8 hours) and to avoid eating for approximately 1 hour after dosing.

The primary objective of this proof-of-concept study was to determine the effect of treatment with the L-arginine salt of Compound 1 in changing 3-component Mayo Clinic score (score ranging from 0 to 9, including stool frequency, rectal bleeding and findings on endoscopy) at 12 weeks.

Secondary endpoints were the proportion of patients who achieve endoscopic improvement at Week 12; change in 2-component Mayo Score (score ranging from 0 to 6, including rectal bleeding and findings on endoscopy) at Week 12; and change in Total Mayo Score (score ranging from 0 to 12, including stool frequency, rectal bleeding, findings on endoscopy, and physician global assessment) at Week 12.

Exploratory endpoints included change from baseline in lymphocyte counts at Weeks 1, 2, 4, 8, and 12; proportion of patients achieving clinical remission at Week 12; and proportion of patients who achieve clinical response at Week 12.

ANCOVA model, adjusted for current oral corticosteroid use, prior exposure to TNF-alpha antagonists, and baseline value, was used to estimate changes in Mayo Clinic Score. Mantel-Haenszel method (estimated treatment difference by adjusting current oral corticosteroid use and prior exposure to TNF-alpha antagonists) used to estimate proportion difference for dichotomous parameters. Missing individual Mayo subscores impacting efficacy measures were imputed using multiple imputation methodology with observed case analysis for sensitivity. Statistical testing was pre-specified as one-sided with p < 0.025 reflecting conventional statistical significance. Hierarchical closed testing procedure for primary and secondary endpoints at 0.05 alpha level was used.

Patients receiving high dose (2 mg) of the L-arginine salt of Compound 1 achieved the primary and all secondary endpoints with statistical significance.

Relative to placebo, there was a 0.99 improvement in a 3-component Partial Mayo Score (PMS; score ranging from 0 to 9 including stool frequency, rectal bleeding and findings on endoscopy) with the L-arginine salt of Compound 1 at 2 mg at week 12, which was statistically significant (p=0.009). In the low dose (1 mg) group, there was a 0.43 improvement in PMS at week 12 relative to placebo, which was not statistically significant (p=0.146).

Significantly more patients in the L-arginine salt of Compound 1 (2 mg) group achieved endoscopic improvement compared with placebo (41.8% vs. 17.8%, p=0.003). For the 1 mg group, 22.5% of the patients achieved endoscopic improvement (p=0.306).

Relative to placebo, there was a 0.84 improvement in a 2-component Mayo Score (score ranging from 0 to 6, including rectal bleeding and findings on endoscopy) at Week 12, which was statistically significant (p=0.002). In the low dose (1 mg) group, there was a 0.39 improvement relative to placebo, which was not statistically significant (p=0.086).

Relative to placebo, there was a 1.27 improvement in Total Mayo Score (score ranging from 0 to 12, including stool frequency, rectal bleeding, findings on endoscopy, and physician global assessment) at Week 12, (p=0.010) for the 2 mg group. In the low dose (1 mg) group, there was a 0.60 improvement relative to placebo, which was not statistically significant (p=0.128).

In exploratory analyses, the proportion of patients achieving clinical remission, defined by the 3-component Mayo Score, was 33.0% in the L-arginine salt of Compound 1 (2 mg) group compared to 8.1% for placebo group (p < 0.001). For the 1 mg group, 16.0% of the patients achieved clinical remission (p = 0.136)

Remission defined by the 4-component Total Mayo Score was 24.5% and 6.0% for L-arginine salt of Compound 1 and placebo, respectively (p=0.004). Remission for the 1 mg group was 15.4% (p=0.077).

Relative to placebo, there was a 57% reduction in lymphocytes, (p<0.001) for the 2 mg group and a 37% reduction for the 1 mg group, each at 12 weeks.

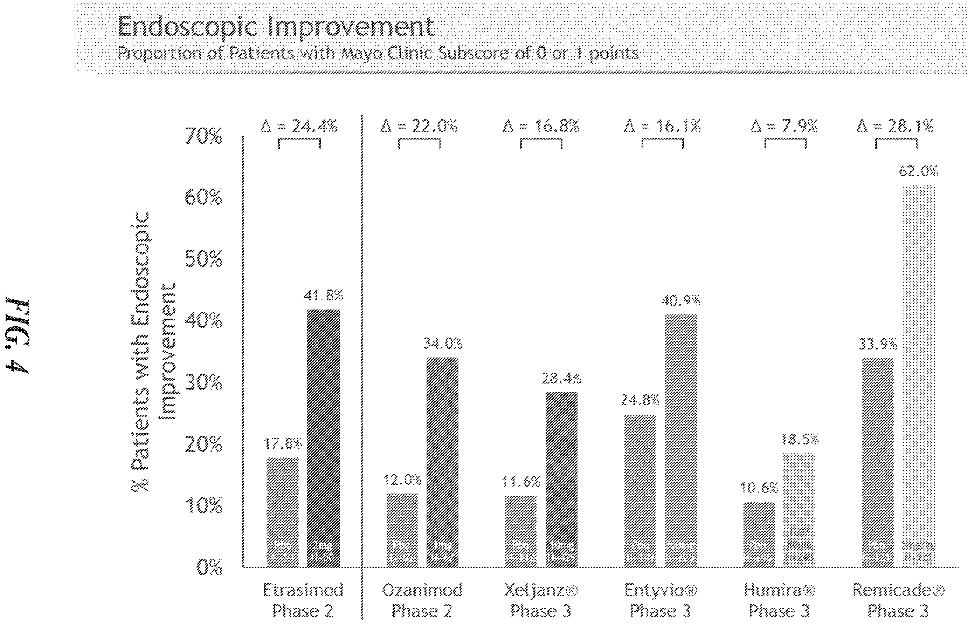

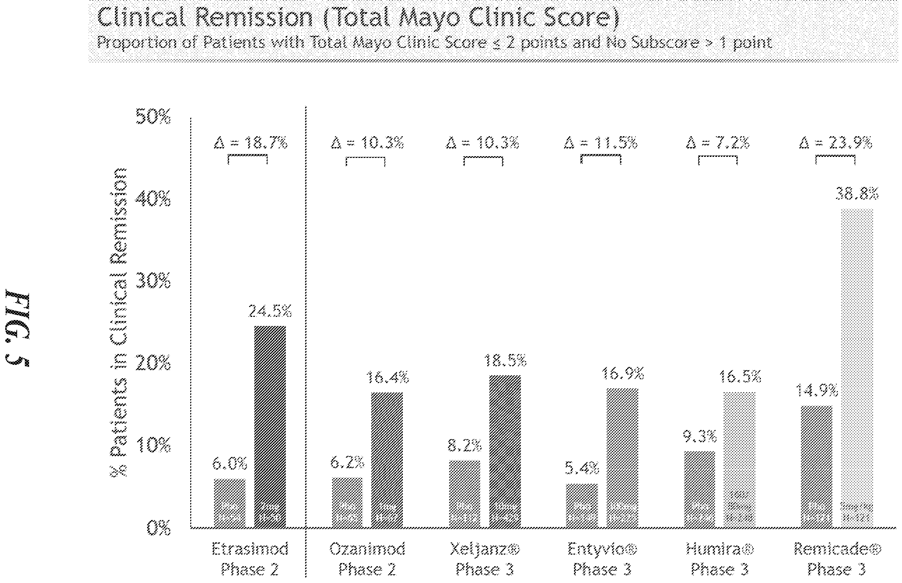

**Figure 4** shows a comparison of percentage of patients with endoscopic improvement for various treatments for ulcerative colitis. **Figure 5** shows a comparison of percentage of patients in clinical remission, which is defined as the proportion of patients with total Mayo score ≤ 2 points and no subscore >1. for various treatments for ulcerative colitis. Note that the definition of remission differed across the studies and that the comparison did not result from direct head-to-head studies

The L-arginine salt of Compound 1 was well tolerated and there were fewer serious adverse events (SAEs) compared to placebo (0% in 2 mg; 5.8% in 1 mg; and 11.1% in placebo).

**TABLE 12**

| | **Placebo (n=54)** | **L-arginine salt of Compound 1** | **L-arginine salt of Compound 1** |
|---|---|---|---|
| | | **1 mg (n=52)** | **2 mg (n=50)** |
| Number (%) of Patients with any TEAE | 27 (50.0) | 31 (59.6) | 28 (56.0) |
| Number (%) of Patients with TEAE Leading to Discontinuation of Study Drug | 0 | 3 (5.8) | 4 (8.0) |
| Number (%) of Patients with Serious TEAE | 6 (11.1) | 3 (5.8) | 0 |
| Number (%) of Deaths of Any Reason | 0 | 0 | 0 |

Impact on heart rate and AV conduction was low throughout the study with no discontinuations from study related to bradycardia or AV block. There were no increases in liver function tests (LFTs) compared to placebo and no reports of macular edema or pulmonary function test abnormalities.

With regard to possible cardiac events, hourly ECGs on Day 1 in both the 1 mg and 2 mg groups showed a mild decrease in heart rate with no mean change in heart rate ≥ 10 bpm in either group at any time point. After Day 1, the mean decrease in heart rate from baseline did not exceed 6 bpm in either dose group through 12 weeks. No serious adverse events related to heart rate changes or AV block were recorded.

There were no increases in liver function tests compared to placebo; no reports of macular edema; and no reports of abnormal pulmonary function tests.

The adverse events in infections and infestations were assessed as mild or moderate by investigators. No severe or life-threatening infection occurred. The majority of the adverse events were upper respiratory tract infections.

### EXAMPLE 4

Patients were randomized to receive etrasimod (the L-arginine salt of Compound 1) 1 mg (n=52), etrasimod 2 mg (n=50), or placebo (n=54). Clinical and endoscopic outcomes were evaluated at baseline and week 12 using Mayo Clinic scores. The modified Mayo Clinic score (modified MCS) included endoscopic, rectal bleeding (RB), and stool frequency (SF) subscores. Remission was defined as an endoscopic subscore ≤1 (with the absence of friability), RB and SF scores ≤1, and a SF decrease from baseline of ≥1. Response was defined as clinical remission or decrease in modified MCS of ≥ 2 points and ≥30% decrease from baseline, with either an RB decrease of ≥1 or RB score of ≤1. Endoscopic improvement was defined as a subscore ≤1. Fecal calprotectin (FC) and C-reactive protein (CRP) were measured at baseline and weeks 4, 8, and 12 from stool and blood samples, respectively. The treatment effect trend over time was analyzed with a mixed-model for repeat measures (1-sided P values) with current oral corticosteroid use, prior exposure to TNF-α antagonists, treatment, week, and treatment-by-week interaction as factors, and baseline value as a covariate. Comparisons between subgroups were assessed with a Wilcoxon rank-sum test (2-sided P values). Analysis of correlation between variables was conducted using the Spearman's rank coefficient (2-sided P values).

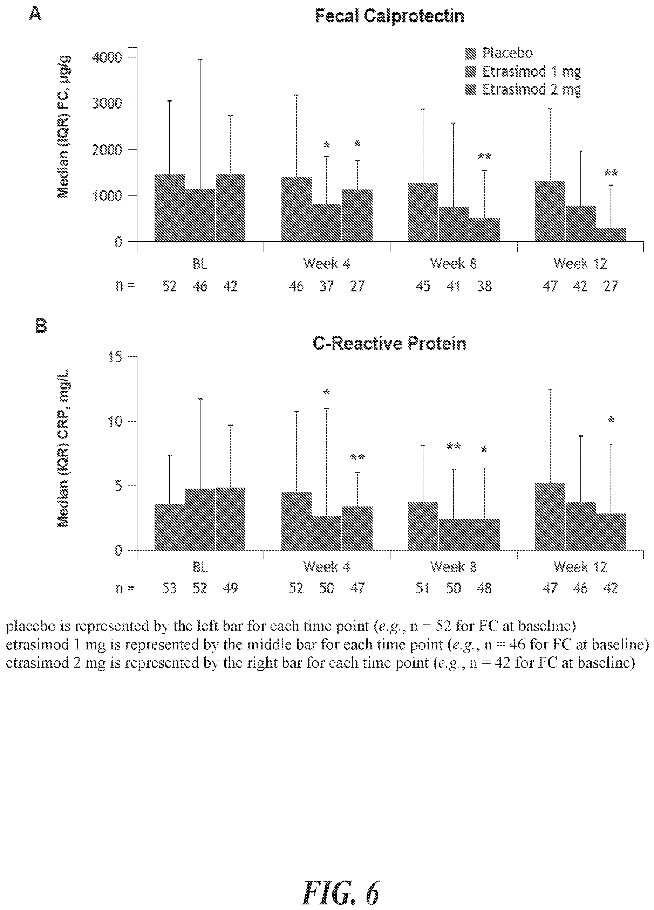

Patients who received etrasimod 2 mg had significant decreases in FC and CRP compared with placebo across the study (0.42 [P<0.001] and 0.70 [P=0.01], respectively). Patients who received etrasimod 1 mg had a significant decrease in CRP compared with placebo across the study (0.72 [*P*=0.02]), and a decrease in FC versus placebo that did not reach statistical significance (0.71 [*P*=0.08]). Decreases in FC and CRP levels in patients who received etrasimod 2 mg versus placebo were first observed at week 4 and were sustained through week 12 (see **Figure 6****).**

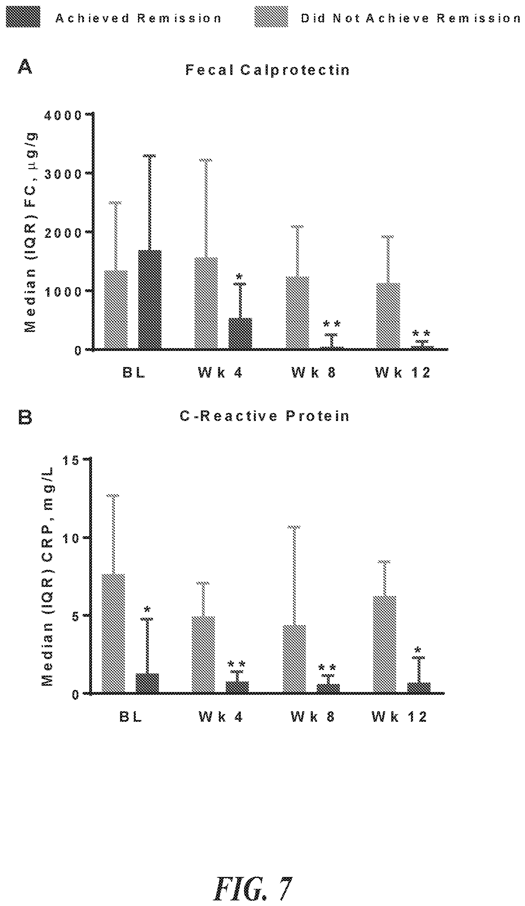

Patients who received etrasimod 2 mg and achieved remission at week 12 had significantly lower levels of FC at all post-baseline time points compared with patients who did not achieve remission (see **Figure 7A****).** The median FC at week 12 for patients who achieved remission versus those who did not achieve remission was 62.0 versus 1129 µg/g (*P*<0.001). Patients who received etrasimod 2 mg and achieved remission at week 12 had significantly lower levels of CRP at all time points, including at baseline, compared with patients who did not achieve remission (see **Figure 7B****).** High CRP level at baseline was associated with lower remission rates during induction therapy.

There was good correlation between clinical and endoscopic outcomes with FC at week 12 (rho 0.71 and 0.70, respectively, *P*<0.001 for both). In the etrasimod 2 mg group, strong correlations (>0.5) were observed between modified MCS and FC, between endoscopic outcomes and FC, and between modified MCS and CRP. Moderate correlations (0.3 to ≤0.5) were observed between endoscopic outcomes and CRP **(Table 13).** In the etrasimod 2 mg group, there was a strong correlation between CRP and FC.

**TABLE 13**

| **Correlation Between Clinical and Endoscopic Disease Activity, Fecal Calprotectin, and C-Reactive Protein at Week 12** | | | | |
|---|---|---|---|---|
| **Variable Compared** | | **Etrasimod 1 mg** | **Etrasimod 2 mg** | **Placebo** |
| **Modified MCS vs FC** | | n=42 | n=37 | n=47 |
| | Spearman coefficient | 0.40 | 0.71 | 0.46 |
| | | *P*=0.009 | *P*<0.001 | *P*=0.001 |
| **Endoscopic subscore vs FC** | | n=42 | n=37 | n=47 |
| | Spearman coefficient | 0.29 | 0.70 | 0.32 |
| | | *P*=0.067 | *P*<0.001 | *P*=0.030 |
| **Modified MCS vs CRP** | | n=46 | n=42 | n=47 |
| | Spearman coefficient | 0.42 | 0.55 | 0.45 |
| | | *P*=0.003 | *P*<0.001 | *P*=0.002 |
| **Endoscopic subscore vs CRP** | | n=46 | n=42 | n=47 |
| | Spearman coefficient | 0.36 | 0.44 | 0.40 |
| | | *P=*0.014 | *P*=0.003 | *P*=0.005 |
| **CRP vs FC** | | n=42 | n=34 | n=46 |
| | Spearman coefficient | 0.30 | 0.61 | 0.35 |
| | | *P*=0.050 | *P*=0.001 | *P*=0.016 |
| CRP, C-reactive protein; FC, fecal calprotectin; MCS, Mayo Clinic score. | | | | |

FC and CRP are surrogate markers of clinical and endoscopic response in patients with ulcerative colitis receiving etrasimod. Patients receiving etrasimod 2 mg had a significant decrease in FC and CRP during treatment.

## Claims

1. The compound (*R*)-2-(7-(4-cyclopentyl-3-(trifluoromethyl)benzyloxy)-1,2,3,4-tetrahydrocyclopenta[b]indol-3-yl)acetic acid (Compound 1), or a pharmaceutically acceptable salt, solvate or hydrate thereof,
for use in a method for the treatment of a sphingosine 1-phosphate subtype 1 (S1P₁) receptor-associated disorder in an individual in need thereof, wherein the S1P₁ receptor-associated disorder is ulcerative colitis, comprising:
administering Compound 1, or a pharmaceutically acceptable salt, solvate or hydrate thereof, to the individual for a first time period, wherein the first time period of treatment is 4 weeks of treatment;
wherein the dose is in an amount equivalent to 2 mg of Compound 1;
wherein the dose of Compound 1, or a pharmaceutically acceptable salt, hydrate, or solvate thereof is administered once daily to the individual;
wherein the Compound 1, or a pharmaceutically acceptable salt, hydrate, or solvate thereof, is administered orally; and
continuing administration of Compound 1 for a second time period if the individual achieves at least 1, 2, 3, 4 ,5, 6, 7, 8, 9, 10, 11, 12, 13, 14, 15, 16, 17, 18, 19, 20, 21, 22, 23, 24, 25, 30, 35, 40, 45, 50, 55, 60, 65, 70, 75, 80, 85, 90, or 95% reduction in fecal calprotectin by the first time period.

2. The compound for use of claim 1, wherein the individual was previously administered at least one agent selected from: a TNF antagonist, an integrin antagonist, and an immunosuppressive agent.

3. The compound for use of any one of the preceding claims, wherein the ulcerative colitis is moderately to severely active ulcerative colitis.

4. The compound for use of any one of the preceding claims, wherein the Compound 1, or a pharmaceutically acceptable salt, hydrate, or solvate thereof, is formulated as a capsule or tablet suitable for oral administration.

5. The compound for use of any one of the preceding claims, wherein the Compound 1, or a pharmaceutically acceptable salt, hydrate, or solvate thereof, is selected from:
Compound 1;
a calcium salt of Compound 1; and
an L-arginine salt of Compound 1.

6. The compound for use of any one of the preceding claims, wherein the Compound 1, or a pharmaceutically acceptable salt, hydrate, or solvate thereof, is an L-arginine salt of Compound 1.

7. The compound for use of any one of the preceding claims, wherein the Compound 1, or a pharmaceutically acceptable salt, hydrate, or solvate thereof, is an anhydrous, non-solvated crystalline form of an L-arginine salt of Compound 1.

8. The compound for use of any one of claims 1 to 5, wherein the Compound 1, or a pharmaceutically acceptable salt, hydrate, or solvate thereof, is an anhydrous, non-solvated crystalline form of Compound 1.

## Patentansprüche

1. Verbindung (*R*)-2-(7-(4-Cyclopentyl-3-(trifluormethyl)benzyloxy)-1,2,3,4-tetrahydrocyclopenta[*b*]indol-3-yl)essigsäure (Verbindung 1) oder ein pharmazeutisch verträgliches Salz, Solvat oder Hydrat davon
zur Verwendung in einem Verfahren zur Behandlung einer mit dem Sphingosin-1-Phosphat-Subtyp-1 (S1P₁)-Rezeptor assoziierten Erkrankung bei einer Person, die einer solchen Behandlung bedarf, wobei die mit dem S1P₁-Rezeptor assoziierte Erkrankung Colitis ulcerosa ist, umfassend:
Verabreichung der Verbindung 1 oder eines pharmazeutisch verträglichen Salzes, Solvats oder Hydrats davon an die Person über einen ersten Zeitraum, wobei der erste Behandlungszeitraum 4 Wochen beträgt;
wobei die Dosis einer Menge entspricht, die 2 mg der Verbindung 1 entspricht;
wobei die Dosis der Verbindung 1 oder eines pharmazeutisch verträglichen Salzes, Hydrats oder Solvats davon der Person einmal täglich verabreicht wird;
wobei die Verbindung 1 oder ein pharmazeutisch verträgliches Salz, Hydrat oder Solvat davon oral verabreicht wird; und
Fortsetzung der Verabreichung von Verbindung 1 für einen zweiten Zeitraum, wenn die Person mindestens eine 1-, 2-, 3-, 4-, 5-, 6-, 7-, 8-, 9-, 10-, 11-, 12-, 13-, 14-, 15-, 16-, 17-, 18-, 19-, 20-, 21-, 22-, 23-, 24-, 25-, 30-, 35-, 40-, 45-, 50-, 55-, 60-, 65-, 70-, 75-, 80-, 85-, 90- oder 95%ige Senkung des fäkalen Calprotectins bis zum ersten Zeitabschnitt erreicht.

2. Verbindung zur Verwendung gemäß Anspruch 1, wobei der Person zuvor mindestens ein Mittel verabreicht wurde, ausgewählt aus: einem TNF-Antagonisten, einem Integrin-Antagonisten und einem Immunsuppressivum.

3. Verbindung zur Verwendung gemäß einem der vorstehenden Ansprüche, wobei es sich bei der Colitis ulcerosa um eine mäßig bis schwer aktive Colitis ulcerosa handelt.

4. Verbindung zur Verwendung gemäß einem der vorstehenden Ansprüche, wobei die Verbindung 1 oder ein pharmazeutisch verträgliches Salz, Hydrat oder Solvat davon als Kapsel oder Tablette formuliert ist, die zur oralen Verabreichung geeignet ist.

5. Verbindung zur Verwendung gemäß einem der vorstehenden Ansprüche, wobei die Verbindung 1 oder ein pharmazeutisch verträgliches Salz, Hydrat oder Solvat davon ausgewählt ist aus:
Verbindung 1;
einem Calciumsalz der Verbindung 1; und
einem L-Arginin-Salz der Verbindung 1.

6. Verbindung zur Verwendung gemäß einem der vorstehenden Ansprüche, wobei die Verbindung 1 oder ein pharmazeutisch verträgliches Salz, Hydrat oder Solvat davon ein L-Arginin-Salz der Verbindung 1 ist.

7. Verbindung zur Verwendung gemäß einem der vorstehenden Ansprüche, wobei die Verbindung 1 oder ein pharmazeutisch verträgliches Salz, Hydrat oder Solvat davon eine wasserfreie, nicht solvatisierte kristalline Form eines L-Arginin-Salzes der Verbindung 1 ist.

8. Verbindung zur Verwendung gemäß einem der Ansprüche 1 bis 5, wobei die Verbindung 1 oder ein pharmazeutisch verträgliches Salz, Hydrat oder Solvat davon eine wasserfreie, nicht solvatisierte kristalline Form der Verbindung 1 ist.

## Revendications

1. Composé (*R)*-2-(7-(4-cyclopentyl-3-(trifluorométhyl)benzyloxy)-1,2,3,4-tétrahydrocyclopenta[*b*]indol-3-yl)acide acétique (composé 1), ou sel, solvate ou hydrate pharmaceutiquement acceptable de celui-ci,
pour une utilisation dans une méthode pour le traitement d'un trouble associé au récepteur de la sphingosine 1-phosphate de sous-type 1 (S1P₁) chez un individu qui en a besoin, dans lequel le trouble associé au récepteur de la S1P₁ est une rectocolite hémorragique, comportant :
l'administration du composé 1, ou d'un sel, solvate ou hydrate pharmaceutiquement acceptable de celui-ci, à l'individu pendant une première période, dans lequel la première période de traitement est de 4 semaines de traitement ;
dans lequel la dose est dans une quantité équivalente à 2 mg de composé 1 ;
dans lequel la dose du composé 1, ou d'un sel, hydrate ou solvate pharmaceutiquement acceptable de celui-ci est administrée une fois par jour à l'individu ;
dans lequel le composé 1, ou un sel, hydrate ou solvate pharmaceutiquement acceptable de celui-ci, est administré par voie orale ; et
la poursuite de l'administration du composé 1 pendant une deuxième période si l'individu atteint au moins 1, 2, 3, 4, 5, 6, 7, 8, 9, 10, 11, 12, 13, 14, 15, 16, 17, 18, 19, 20, 21, 22, 23, 24, 25, 30, 35, 40, 45, 50, 55, 60, 65, 70, 75, 80, 85, 90 ou 95 % de réduction de la calprotectine fécale lors de la première période.

2. Composé pour une utilisation
selon la revendication 1, dans lequel au moins un agent choisi parmi : un antagoniste du TNF, un antagoniste de l'intégrine et un agent immunosuppresseur a été précédemment administré à l'individu.

3. Composé pour une utilisation
selon l'une quelconque des revendications précédentes, dans lequel la rectocolite hémorragique est une rectocolite hémorragique modérément à sévèrement active.

4. Composé pour une utilisation
selon l'une quelconque des revendications précédentes, dans lequel le composé 1, ou
un sel, hydrate ou solvate pharmaceutiquement acceptable de celui-ci est formulé sous la forme d'une capsule ou d'un comprimé adapté pour une administration orale.

5. Composé pour une utilisation
selon l'une quelconque des revendications précédentes, dans lequel le composé 1, ou un sel, hydrate ou solvate pharmaceutiquement acceptable de celui-ci est choisi parmi :
le composé 1 ;
un sel de calcium du composé 1 ; et
un sel de L-arginine du composé 1.

6. Composé pour une utilisation
selon l'une quelconque des revendications précédentes, dans lequel le composé 1, ou
un sel, hydrate ou solvate pharmaceutiquement acceptable de celui-ci est un sel de L-arginine du composé 1.

7. Composé pour une utilisation
selon l'une quelconque des revendications précédentes, dans lequel le composé 1, ou
un sel, hydrate ou solvate pharmaceutiquement acceptable de celui-ci est une forme cristalline anhydre, non solvée d'un sel de L-arginine du composé 1.

8. Composé pour une utilisation
selon l'une quelconque des revendications 1 à 5, dans lequel le composé 1, ou un sel, hydrate ou solvate pharmaceutiquement acceptable de celui-ci, est une forme cristalline anhydre, non solvée du composé 1.
